# EUROPÄISCHE PATENTSCHRIFT

(11) **EP 0 710 248 B1**
(45) Veröffentlichungstag und Bekanntmachung des Hinweises auf die Patenterteilung: **29.09.1999**
(21) Anmeldenummer: 94925364.5
(22) Anmeldetag: 19.07.1994
(51) Int. Cl.: C07K 14/52, C12N 15/07, C12N 15/19, A61K 38/22

(54) **MELANOM-INHIBIERENDES PROTEIN**
MELANOMA-INHIBITING PROTEIN
PROTEINE D'INHIBITION DE MELANOMES

(30) Priorität: 20.07.1993 DE 4324247
(43) Veröffentlichungstag der Anmeldung: 08.05.1996
(60) Teilanmeldung: 99101554.6
(73) Patentinhaber: Roche Diagnostics GmbH, 68298 Mannheim (DE)
(72) Erfinder: BOGDAHN, Ulrich, D-97078 Würzburg (DE); BÜTTNER, Reinhard, D-93090 Bach (DE); KALUZA, Brigitte, D-83670 Bad Heilbrunn (DE)
(86) Internationale Anmeldenummer: EP9402369
(87) Internationale Veröffentlichungsnummer: WO9503328

(56) Entgegenhaltungen:
- CANCER RESEARCH, Bd.49, 1989 Seiten 5358 - 5363 U.BOGDAHN ET AL. 'Autocrine Tumor Cell Growth-inhibiting Activities from Human Malignant Melanoma'
- MELANOMA RES., Bd.2, Nr.5-6, 1992 Seiten 327 - 36 R.APFEL ET AL. 'Purification and analysis of growth regulating proteins secreted by a human melanoma cell line'

## Beschreibung

Die Erfindung betrifft ein Melanom-inhibierendes Protein (MIA), eine dafür codierende Nukleinsäure, ein Verfahren zur Gewinnung und zum Nachweis dieses Proteins sowie dessen Verwendung zur Herstellung eines Therapeutikums.

Die Regulation des Zellwachstums wird sowohl durch positiv als auch negativ wirkende Faktoren kontrolliert. Zu den positiv wirkenden Faktoren zählen die bekannten Wachstumsfaktoren wie z. B. epidermal growth factor (EGF), platelet derived growth factor (PDGF), Insulin und Somatomedine. Zu den negativ, d. h. inhibitorisch wirkenden Faktoren gehören neben dem TGF-β, der sowohl als Wachstumsstimulator als auch als Wachstumsinhibitor wirken kann (Roberts et al., Proc. Natl. Acad. Sci. 82 (1985), 119 - 123) die endogenen, tumorinhibierenden Faktoren aus Dickdarmkarzinomzellen (Levine et al, Cancer Research 45 (1985), 2248 - 2254), Melanomen (Bogdahn et al., Cancer Research 49 (1989), 5358 - 5363) sowie aus gesunden epithelialen Zellen von Brustdrüsen der Ratte (Ethier et al., J. Cell. Phys. 142 (1990), 15 - 20).

Durch Störung dieses Regulationssystems, wie z. B. durch Überproduktion von positiv wirkenden Wachstumsfaktoren oder eine verminderte Abhängigkeit veränderter Zellen von diesen Wachstumsfaktoren (Rodeck et al., International Journal of Cancer 40 (1987), 687 - 690) wird es Tumorzellen ermöglicht, sich unkontrolliert zu vermehren. Die oben genannten tumorinhibierenden Faktoren aus verschiedenen Tumorgeweben stellen interessante Verbindungen dar, um in dieses gestörte Regulationssystem therapeutisch eingreifen zu können. Für eine solche therapeutische Verwendung müssen diese Faktoren in großer Menge und reproduzierbarer Reinheit zur Verfügung gestellt werden können. Bislang sind jedoch für die meisten dieser Faktoren lediglich angereicherte Fraktionen aus Zellysaten beschrieben, die auf Grund ihrer komplexen und zum Teil unbekannten Zusammensetzung und der damit verbundenen nicht reproduzierbaren Herstellbarkeit für einen therapeutischen Einsatz nicht geeignet sind.

Grundlage der Erfindung ist ein neues Melanom-inhibierendes Protein (im weiteren als MIA-Protein oder MIA bezeichnet), welches das Wachstum der Zellinien HTZ 19-dM und ATCC CRL 1424 inhibiert und
a) codiert wird von der in SEQ ID NO 1 für das reife Protein bzw. für das Protein mit N-terminaler Prä-Sequenz gezeigten DNA-Sequenz, oder der in SEQ ID NO 3 gezeigten genomischen Sequenz,
b) codiert wird von DNA-Sequenzen, die mit den in SEQ ID NO 1 oder 3 gezeigten DNA-Sequenzen oder Fragmenten dieser DNA-Sequenzen im DNA-Bereich, der für das reife Protein codiert, hybridisieren.

Unter einer Wachstuminhibierung ist eine antiproliferative Wirkung zu verstehen. Dabei wird das Wachstum der Zellen bei Zusatz des MIA-Proteins zum Kulturmedium deutlich verlangsamt. Eine hierfür geeignete Konzentration ist beispielsweise O,1 µg MIA-Protein/ml Kulturmedium. Höhere oder geringere Konzentrationen des MIA-Proteins sind jedoch ebenfalls geeignet zur Wachstumsinhibierung, die je nach Konzentration stärker oder geringer beobachtet wird.

Die Eigenschaften eines solchen Proteins sind durch die Erfinder in Cancer Research 49 (1989), 5358 - 5363, Cancer Research 50 (1990), 6981 - 6986, Melanoma Research 2 (1992), 327 - 336 beschrieben. Ein nacharbeitbares Verfahren zur Herstellung dieses Proteins ist jedoch in diesen Publikationen nicht angegeben. Das Protein ist aus der humanen Melanomazellinie HTZ 19-dM, die bisher der Öffentlichkeit noch nicht zugänglich war, erhältlich. Diese Zellinie wurde von einem metastasierenden malignen Melanom abgeleitet und in definiertem serumfreien Kulturmedium (50 % Dulbecco's minimal essential medium, 50 % F-12) mit 0,8 mmol/l L-Glutamin, nichtessentiellen Aminosäuren, 10 µg/ml Transferrin, 30 nmol/l Natriumselenit und 4 µg/ml Gentamicin als Monolayer-Kultur unter Standard-Kulturbedingungen kultiviert. Die Zellinie wurde am 23.06.93 bei der Deutschen Sammlung für Mikroorganismen und Zellkulturen GmbH in Braunschweig hinterlegt (DSM ACC 2133). Sie ist ein weiterer Gegenstand der Erfindung. Aus dem Kulturüberstand dieser Zellinie kann das erfindungsgemäße Protein über eine gelchromatographische Isolierung einer Proteinfraktion einer Größe von ca. 11 kD und anschliessende Auf reinigung dieser Fraktion über eine reverse phase HPLC gewonnen werden.

Das Protein kann über seine DNA-Sequenz und die davon abgeleitete Aminosäuresequenz definiert werden. Das MIA-Protein kann in natürlichen allelischen Variationen, die sich von Individuum zu Individuum unterscheiden können, vorkommen (z.B. SEQ ID NO:24). Solche Variationen der Aminosäuren sind in der Regel Aminosäureaustausche. Es kann sich aber auch um Deletionen, Insertionen oder Additionen von Aminosäuren zur Gesamtsequenz handeln. Das erfindungsgemäße MIA Protein kann - sowohl in Umfang und Art abhängig von der Zelle und dem Zelltyp, in dem es exprimiert wird - glycosyliert oder nicht glycosyliert sein.

Das erfindungsgemäße Protein kann auch rekombinant hergestellt werden. Bei der rekombinanten Herstellung in Prokaryonten wird nichtglykosyliertes MIA-Protein erhalten. Mit Hilfe der durch die Erfindung bereitgestellten Nukleinsäuresequenzen ist es möglich, in Genomen von beliebigen Zellen (z. B. außer in Humanzellen auch in Zellen anderer Säugetiere) nach dem MIA-Gen oder seinen Varianten zu suchen, diese zu identifizieren und das gewünschte für das MIA-Protein codierende Gen zu isolieren. Derartige Verfahren und die hierfür geeigneten Hybridisierungsbedingungen sind dem Fachmann bekannt und beispielsweise bei J. Sambrook, E.F. Fritsch und T. Maniatis, Molecular cloning, Cold Spring Harbor Laboratory, 1989 und B.D. Hames, S.G. Higgins, Nucleic acid hybridisation - a practical approach (1985) IRL Press, Oxford, England, beschrieben. Üblicherweise werden hierbei die dort beschriebenen Standardprotokolle für die Experimente verwendet.

Durch die Anwendung der rekombinanten DNA-Technik ist es möglich, eine Vielzahl von MIA-Protein-Derivaten herzustellen. Derartige Derivate können beispielsweise modifiziert sein in einzelnen oder mehreren Aminosäuren durch Substitution, Deletion oder Addition. Die Derivatisierung kann beispielsweise über site directed mutagenesis erfolgen. Derartige Variationen sind für einen Fachmann ohne weiteres durchführbar (J. Sambrook, B.D. Hames, Loc. Lit.). Es muß lediglich sichergestellt sein, daß die charakteristischen Eigenschaften des MIA-Proteins (Inhibition der genannten Zellinien) erhalten bleiben.

Ein weiterer Gegenstand der Erfindung ist deshalb ein MIA-Protein, welches
a) das Produkt einer prokaryontischen oder eukaryontischen Expression einer exogenen DNA ist,
b) codiert wird von der in SEQ ID NO 1 für das reife Protein bzw. für das Protein mit N-terminaler Prä-Sequenz gezeigten DNA-Sequenz, der in SEQ ID NO 3 gezeigten genomischen Sequenz,
c) codiert wird von DNA-Sequenzen, die mit den in SEQ ID NO 1 oder 3 gezeigten DNA-Sequenzen oder Fragmenten dieser DNA-Sequenzen im DNA-Bereich, der für das reife Protein codiert, hybridisieren, oder
d) codiert wird von DNA-Sequenzen, die ohne die Degeneration des genetischen Codes mit den in b) bis c) definierten Sequenzen hybridisieren würden und für ein Polypeptid mit Aminosäuresequenz codieren.

Bevorzugt ist ein Protein, welches codiert wird von den Nukleotiden 40 - 432 oder 112 bis 432 aus SEQ ID NO 1 oder von DNA-Sequenzen, die aufgrund der Degeneration des genetischen Codes für ein Polypeptid mit derselben Aminosäuresequenz codieren würden.

Das MIA-Protein aus HTZ 19-dM weist ein Molekulargewicht von ca. 11 kD auf, ist stabil gegen Hitze (3 Minuten bei 100° C) und sensitiv gegenüber Proteasen, wie z. B. Trypsin.

Gegenstand der Erfindung ist eine Nukleinsäure, welche für ein MIA-Protein codiert und ausgewählt ist aus der Gruppe
a) der in SEQ ID NO 1 und 3 gezeigten DNA-Sequenzen oder den komplementären Sequenzen,
b) Nukleinsäuren-Sequenzen, die mit einer der Sequenzen von a) hybridisieren,
c) Nukleinsäuren-Sequenzen, die ohne die Degeneration des genetischen Codes mit einer der in a) oder b) genannten Sequenzen hybridisieren würden.

Ein weiterer Gegenstand der Erfindung sind melanom-inhibierende Proteine aus Säugerzellen, wie z.B. Maus, Ratte, Rind, Schaf, welche in im wesentlichen analoger Weise das Wachstum der Zellinien HTZ19-dM und ATCC CRL 1424 inhibieren, wie das humane MIA-Protein.

Diese dem humanen MIA-Protein analogen Proteine können dadurch erhalten werden, daß mit einer Hybridisierungsprobe, welche für humanes MIA codierende Sequenzen enthält, eine cDNA-Bibliothek des entsprechenden Säugetiers nach dem Fachmann geläufigen Methoden gescreent wird und über den Sequenzvergleich mit der DNA und Proteinsequenz für humanes und murines MIA (SEQ ID NO:1-5) das entsprechende codierende Segment identifiziert wird.

Ein bevorzugter Gegenstand der Erfindung ist das murine MIA-Protein und die hierfür codierende Nukleinsäuresequenz (SEQ ID NO:4). Das murine Protein wird codiert von den Nukleotiden 110 - 499 oder 179 - 499 von SEQ ID NO:4.

Mit Hilfe dieser Nukleinsäuren kann das erfindungsgemäße Protein in reproduzierbarer Weise in großen Mengen gewonnen werden. Zur Expression in prokaryontischen oder eukaryontischen Organismen, wie z.B. prokaryontischen Wirtszellen oder in eukaryontischen Wirtszellen, wird die Nukleinsäure nach dem Fachmann geläufigen Verfahren in geeignete Expressionsvektoren integriert. Vorzugsweise enthält ein solcher Expressionsvektor einen regulierbaren/induzierbaren Promotor. Zur Expression werden diese rekombinanten Vektoren dann nach bekannten Verfahren in geeignete Wirtszellen, wie z. B. E.coli, als prokaryontische Wirtszellen oder Saccharomyces cerevisiae, Teratocarcinoma-Zellinie PA-1 sc 9117 (Büttner et al., Mol. Cell. Biol. 11, 1991, 3573 - 3583), Insektenzellen, CHO- oder COS-Zellen als eukaryontische Wirtszellen eingeführt und die transformierten bzw. transduzierten Wirtszellen unter Bedingungen kultiviert, die eine Expression des heterologen Gens ermöglichen. Die Isolierung des Proteins kann aus der Wirtszelle oder dem Kulturüberstand der Wirtszelle nach bekannten Verfahren erfolgen. Derartige Verfahren sind beispielsweise bei Ausubel I., Frederick M., Current Protocols in Mol. Biol. (1992), John Wiley and Sons, New York, beschrieben. Es kann auch eine in vitro Reaktivierung des Proteins erforderlich sein.

Vorzugsweise wird zur rekombinanten Herstellung des erfindungsgemäßen Proteins eine DNA mit den Nukleotiden 40 - 432 oder 112 - 432 (codierende Sequenz) aus SEQ ID NO 1 (cDNA) oder die genomische DNA gemäß SEQ ID NO 3 verwendet.

Ein weiterer Gegenstand der Erfindung ist ein Verfahren zur Gewinnung eines MIA-Proteins durch Isolierung aus dem Kulturüberstand der Melanomzelline HTZ 19-dM über eine gelchromatographische Auftrennung und Aufreinigung einer Fraktion, die einem Molekulargewicht von ca. 11 kD (SDS-PAGE, nicht reduziert) entspricht, über reverse phase HPLC. Auf diese Weise können ca. 0,2 µg/l Kulturüberstand erhalten werden.

In einer bevorzugten Ausführungsform wird das natürliche MIA-Protein bei der Isolierung/Aufreinigung einer Säurebehandlung unterworfen. Dadurch kann die MIA-Aktivität gesteigert werden. Vorteilhaft ist ein pH-Wert um ca. 2, als Säure ist beispielsweise Essigsäure geeignet.

Der Nachweis von transformierten bzw. transduzierten Wirtszellen, welche das MIA-Protein rekombinant produzieren, sowie die Aufreinigung des Proteins erfolgen vorzugsweise über Antikörper, die an dieses Protein binden. Derartige Antikörper sind mit Hilfe des erfindungsgemäßen Proteins als Antigen oder Immunogen in einfacher Weise nach bekannten Verfahren erhältlich.

Ein weiterer Gegenstand der Erfindung ist daher die Verwendung des erfindungsgemäßen Proteins mit Melanom-inhibierender Aktivität zur Herstellung von Antikörpern, die an dieses Protein binden.

Dazu werden hierzu üblicherweise verwendete Tiere, wie insbesondere Schafe, Kaninchen oder Mäuse mit dem erfindungsgemäßen Protein immunisiert und anschließend das Antiserum der immunisierten Tiere nach bekannten Verfahren gewonnen oder Milzzellen der immunisierten Tiere mit immortalen Zellen, wie z. B. Myelomzellen, gem. dem Verfahren von Köhler und Milstein (Nature 256 (1975), 495 - 497) fusioniert. Von den so erhaltenen Hybridomzellen werden dann solche ausgewählt und kloniert, welche einen monoklonalen Antikörper gegen das MIA-Protein produzieren. Die so erhaltenen monoklonalen oder polyklonalen Antikörper können für eine immunadsorptive Reinigung des Melanom-inhibierenden Proteins an ein Trägermaterial, wie z. B. Cellulose, gebunden werden. Weiterhin können derartige Antikörper für einen Nachweis des MIA-Proteins in Proben, wie etwa Gewebeschnitten oder Körperflüssigkeiten, verwendet werden.

Ein weiterer Gegenstand der Erfindung sind daher Antikörper gegen das MIA-Protein, welche erhältlich sind durch Immunisierung eines Tieres mit einem MIA-Protein und Gewinnung der Antikörper aus dem Serum oder Milzzellen der immunisierten Tiere.

Es hat sich gezeigt, daß das MIA-Protein nicht nur auf Melanomzellen sondern auch in geringerem Umfang auf andere Tumorzellen, wie z. B. Glioblastomzellen, Neuroblastome, small cell lung cancer und neuroektodermale Tumoren eine hemmende Wirkung durch Hemmung der DNA-Synthese (³H-Thymidin-Einbau (Coligan J. E., Kruisbeek A. M., Margulies D. H., Shevach E. M., Strober W., Current Protocols Immunology, NIH Monograph, J. Wiley and Sons, New York, 1992)), Hemmung einer Tumorkoloniebildung im Soft-Agar oder im Tumorstammzellen-Assay (Schlag P., Flentje D., Cancer Treatment Rev. 11: Suppl. A: 131 - 137, 1984) aufweist. Das Wachstum von normalen, nichtentarteten Zellen wird dagegen nicht inhibiert. Dabei wirkt dieses Protein bereits in sehr geringen Konzentrationen (Nanogramm-Bereich). Daher eignet sich dieses Protein für die Herstellung eines Therapeutikums zur Tumortherapie. Insbesondere ist ein solches Therapeutikum zur Therapie von malignen Melanomen, malignen Gliomen, Bronchial-Karzinomen (insbesondere kleinzelliges Bronchialkarzinom, SCLC) und Neuroblastomen geeignet.

Es hat sich weiterhin gezeigt, daß das Melanom-inhibierende Protein die Interleukin 2-abhängige bzw. Phytohämagglutinin-induzierte Proliferation peripherer Blutlymphozyten supprimiert. Weiterhin wird die Cytotoxizität von T-Lymphozyten reduziert. Das Melanom-inhibierende Protein eignet sich damit auch zur Herstellung eines Therapeutikums, das Anwendung als Immunsuppressivum findet.

Ein weiterer Gegenstand der Erfindung ist daher die Verwendung eines erfindungsgemäßen Proteins zur Herstellung eines Therapeutikums, das Anwendung in der Tumortherapie oder als Immunsuppressivum findet.

Für die genannten therapeutischen Anwendungen wird das erfindungsgemäße Protein ggf. mit den üblicherweise verwendeten Hilfs-, Füll- und/oder Zusatzstoffen zusammen in einer pharmazeutischen Formulierung verarbeitet.

Ein weiterer Gegenstand der Erfindung ist daher eine therapeutische Zusammensetzung, enthaltend ein erfindungsgemäßes Melanom-inhibierendes Protein, ggf. zusammen mit den üblicherweise verwendeten Hilfs-, Füll- und/oder Zusatzstoffen.

Ein weiterer Gegenstand der Erfindung ist die Verwendung von Sequenzen des MIA-Gens, vorzugsweise von für ein Protein mit MIA-Aktivität codierenden Sequenzen oder aktivierende Sequenzen aus dem 5' untranslatierten Bereich, in der Gentherapie und insbesondere zur Herstellung von Arzneimitteln für die Gentherapie.

Gentherapie von somatischen Zellen kann beispielsweise unter Verwendung von retroviralen Vektoren, anderen viralen Vektoren oder durch nicht-viralen Gentransfer erfolgen (zur Übersicht vgl. T. Friedmann, Science 244 (1989) 1275; Morgan 1993, RAC DATA MANAGEMENT Report, June 1993).

Gentherapeutisch geeignete Vektorsysteme sind beispielsweise Retroviren (Mulligan, R.C. (1991) in Nobel Symposium 8: Ethiology of human disease at the DNA level (Lindsten, J. and Pattersun Editors), pages 143 - 189, Raven Press), Adeno Associated Virus (McLughlin, J. Virol. 62 (1988), 1963), Vaccinia Virus (Moss et al., Ann. Rev. Immunol. 5 (1987) 305), Bovine Papilloma Virus (Rasmussen et al., Methods Enzymol. 139 (1987) 642) oder Viren aus der Gruppe der Herpesviren, wie Epstein Barr Virus (Margolskee et al., Mol. Cell. Biol. 8 (1988) 2937) oder Herpes Simplex Virus.

Es sind auch nicht-virale Deliverysysteme bekannt. Hierzu wird üblicherweise "nackte" Nukleinsäure, vorzugsweise DNA, verwendet, oder Nukleinsäure zusammen mit einem Hilfsstoff, wie z.B. mit Transferreagenzien (Liposomen, Dendromere, Polylysin-Transferrin-Konjugate (Wagner, 1990; Felgner et al., Proc. Natl. Acad. Sci. USA 84 (1987) 7413)).

Ein weiteres bevorzugtes gentherapeutisches Verfahren basiert auf der homologen Rekombination. Dabei kann entweder das für das MIA-Protein codierende Gen in einer oder mehreren Kopien in das Genom von somatischen Zellen eingeführt werden und/oder das endogen in den Zellen vorhandene MIA-Gen moduliert, vorzugsweise aktiviert werden.

Verfahren zur homologen Rekombination sind beispielsweise beschrieben in Kucherlapati, Proc. in Nucl. Acids Res. and Mol. Biol. 36 (1989) 301; Thomas et al., Cell 44 (1986) 419-428; Thomas und Capecchi, Cell 51 (1987) 503-512; Doetschman et al., Proc. Natl. Acad. Sci. USA 85 (1988) 8583-8587 und Doetschman et al., Nature 330 (1987) 576-578. Bei diesen Verfahren wird ein Stück DNA, das im Genom an einer bestimmte Stelle integriert werden soll (Genfragment von MIA), an eine "Targeting DNA" gebunden. Die Targeting DNA ist eine DNA, die komplementär (homolog) zu einer Region der genomischen DNA ist (vorzugsweise im oder in der Nähe des MIA-Gens). Wenn zwei homologe Stücke einer Einzelstrang-DNA (z.B. die Targeting DNA und die genomische DNA) in unmittelbare Nähe zueinander kommen, hybridisieren sie und bilden eine Doppelstrang-Helix. Durch ein Rekombinationsereignis kann dann das MIA-Genfragment und die Targeting-DNA ins Genom integriert werden. Diese homologe Rekombination kann sowohl in vitro als auch in vivo (am Patienten) durchgeführt werden.

Vorzugsweise wird eine DNA verwendet, die für ein Protein mit MIA-Aktivität codiert, ein Fragment, welches die MIA-Expression inhibiert (Knock-out-Sequenz), oder ein Fragment, welches in der Lage ist, nach Integration ins Genom einer Zelle die Expression eines Proteins mit MIA-Aktivität in dieser Zelle zu aktivieren. Ein solches Fragment kann zum Beispiel eine Promotor- und/oder Enhancer-Region sein, die heterolog zur entsprechenden MIA-Region ist oder nach Integration in das MIA-Gen das an sich stumme oder gering exprimierte MIA-Gen transkriptional und/oder translatorisch aktiviert.

Mit dieser DNA werden also ein oder mehrere MIA-Gene in die Zielzelle (Targetzelle) neu eingebracht oder das im wesentlichen transkriptional stumme Gen im Genom einer Säugerzelle so aktiviert, daß die Säugerzelle befähigt wird, endogenes MIA-Protein herzustellen. Hierzu wird ein DNA-Konstrukt in das Genom durch homologe Rekombination insertiert, wobei dieses DNA-Konstrukt umfaßt: ein DNA-Regulationselement, welches in der Lage ist, die Expression dieses Gens zu modulieren, vorzugsweise zu stimulieren, wenn es operativ daran gekoppelt ist; und ein oder mehrere DNA-Target-Segmente, welche homolog zu einer Region in diesem Genom sind, die innerhalb oder in der Nähe dieses Gens liegt. Dieses Konstrukt wird in einer Weise ins Genom der Säugerzelle insertiert, daß das regulatorische Segment operativ an das Gen gekoppelt ist, welches für das Protein mit MIA-Aktivität codiert. Vorzugsweise umfaßt das Konstrukt noch amplifizierende Sequenzen, insbesondere wenn Gene, die für ein Protein mit MIA-Aktivität codieren, in die Zelle eingebracht werden.

Für die Einführung der MIA-Gene in die Zielzellen umfaßt das Konstrukt ein Regulationselement, ein oder mehrere MIA-Gene und ein oder mehrere Targetsegmente. Die Targetsegmente sind so ausgewählt, daß sie mit einer geeigneten Region des Genoms hybridisieren, wobei die insertierten exogenen MIA-Gene nach homologer Rekombination exprimiert werden.

Es sind eine Vielzahl von Verfahren bekannt, durch die homologe Rekombination initiiert werden kann. Vorzugsweise erfolgt die homologe Rekombination während der DNA-Replikation oder Mitosis der Zellen. Eine derartige DNA kann zur Herstellung eines Tumortherapeutikums oder zur Herstellung eines homologen oder heterologen MIA-Proteins in einem Wirtsorganismus verwendet werden.

Auf Basis der durch die Erfindung bereitgestellten Nukleinsäure-Sequenzen des MIA-Proteins ist es möglich, einen Test bereitzustellen, mit dem Nukleinsäuren, welche für MIA-Proteine codieren, nachgewiesen werden können. Ein derartiger Nachweis kann beispielsweise in Zellen oder Zell-Lysaten erfolgen. Ein solcher Nachweis kann mittels der Nukleinsäuren-Diagnostik geführt werden. Hierbei wird die zu untersuchende Probe (sample) mit einer Sonde in Kontakt gebracht, welche mit der für das MIA-Protein codierenden Nukleinsäuren-Sequenz hybridisieren würde. Eine Hybridisierung zwischen der Sonde und Nukleinsäuren aus der Probe zeigt das Vorhandensein von exprimierten MIA-Proteinen. Derartige Verfahren sind dem Fachmann bekannt und beispielsweise in der WO 89/06698, EP-A 0 200 362, USP 2915082, EP-A 0 063 879, EP-A 0 173 251, EP-A 0 128 018 beschrieben.

In einer bevorzugten Ausführungsform der Erfindung wird die für ein MIA-Protein codierende Nukleinsäure der Probe vor dem Test amplifiziert, beispielsweise mittels der bekannten PCR-Technik. Üblicherweise wird im Rahmen der Nukleinsäurediagnostik eine derivatisierte (markierte) Nukleinsäuresonde verwendet. Diese Sonde wird mit einer an einen Träger gebundenen denaturierten DNA oder RNA aus der Probe in Kontakt gebracht und hierbei die Temperatur, Ionenstärke, pH-Wert und sonstige Pufferbedingungen - abhängig von der Länge der Nukleinsäureprobe und der daraus resultierenden Schmelztemperatur des zu erwartenden Hybrids - so gewählt, daß die markierte DNA oder RNA an homologe DNA oder RNA binden kann (Hybridisierung, siehe auch J. Mol. Biol. 98 (1975), 503; Proc. Natl. Acad. Sci. USA 76 (1979), 3683). Als Träger geeignet sind Membranen oder Trägermaterialien auf Basis Nitrocellulose (z. B. Schleicher und Schüll, BA 85, Amersham Hybond, C.), verstärkte oder gebundene pulverförmige Nitrocellulose oder mit verschiedenen funktionellen Gruppen (z. B. Nitrogruppe) derivatisierte Nylonmembranen (z. B. Schleicher und Schüll, Nytran; NEN, Gene Screen; Amersham Hybond M.; Pall Biodyne).

Die Detektion von hybridisierender DNA oder RNA erfolgt dann dadurch, daß der Träger nach gründlichem Waschen und Absättigen zur Verhinderung unspezifischer Bindung mit einem Antikörper oder Antikörperfragment inkubiert wird. Der Antikörper oder das Antikörper fragment ist gegen die in der Nukleinsäuresonde bei der Derivatisierung inkorporierte Substanz gerichtet. Der Antikörper wiederum ist markiert. Es kann jedoch auch eine direkt markierte DNA verwendet werden. Nach der Inkubation mit den Antikörpern wird nochmals gewaschen, um nur spezifisch gebundene Antikörperkonjugate nachzuweisen. Die Bestimmung erfolgt dann über die Markierung des Antikörpers oder Antikörperfragments nach an sich bekannten Methoden.

Der Nachweis der MIA-Expression kann durchgeführt werden beispielsweise als:
- in situ-Hybridisierung mit fixierten ganzen Zellen mit fixierten Gewebesabstrichen und isolierten Metaphasen-Chromosomen,
- colony-Hybridisierung (Zellen) und plaque-Hybridisierung (Phagen und Viren),
- Northern-Hybridisierung (RNA-Nachweis),
- Serum-Analytik (z. B. Zelltypanalyse von Zellen in Serum, durch slot-blot-Analyse),
- nach Amplifikation (z.B. PCR-Technik).

Die Erfindung umfaßt daher ein Verfahren zum Nachweis von Nukleinsäuren, welche für ein MIA-Protein codieren, dadurch gekennzeichnet, daß die zu untersuchende Probe mit einer Nukleinsäurensonde inkubiert wird, welche ausgewählt ist aus der Gruppe
a) der in Sequenz ID NO 1 und 3 gezeigten DNA-Sequenzen oder der dazu komplementären Sequenz,
b) Nukleinsäuren, die mit einer der Sequenzen von a) hybridisieren,
die Nukleinsäuresonde mit der Nukleinsäure der Probe inkubiert wird und die Hybridisierung ggf. über einen weiteren Bindepartner von Nukleinsäure der Probe und Nukleinsäuresonde nachgewiesen wird.

MIA ist damit ein wertvoller prognostischer Marker in der Tumordiagnostik (Metastasen, Verlauf).

Die Erfindung wird durch die Sequenzprotokolle in Verbindung mit den folgenden Beispielen und Abbildungen näher erläutert. Dabei bedeuten:
SEQ ID NO 1 - cDNA von humanem MIA mit Prä-Sequenz
SEQ ID NO 2 - Protein
SEQ ID NO 3 - genomische DNA von MIA
SEQ ID NO 4 - cDNA von murinem MIA mit Prä-Sequenz
SEQ ID NO 5 - Protein
SEQ ID NO 6 - Primer
SEQ ID NO 7 - Primer
SEQ ID NO 8 - Klonierungsfragment
SEQ ID NO 9 - Primer
SEQ ID NO 10 - Primer
SEQ ID NO 11 - Adaptor
SEQ ID NO 12 - Adaptor
SEQ ID NO 13 - Fusionsprotein
SEQ ID NO 14 - Fusionsprotein
SEQ ID NO 15 - Primer
SEQ ID NO 16 - Primer
SEQ ID NO 17 - Primer
SEQ ID NO 18 - fusionsfreies MIA zur Expression in E.coli
SEQ ID NO 19 - Primer
SEQ ID NO 20 - Primer
SEQ ID NO 21 - Primer
SEQ ID NO 22 - Primer
SEQ ID NO 23 - Polylinker
SEQ ID NO 24 - genomische DNA von MIA (allelische Variante)

- Fig. 1: zeigt die invasionshemmende Wirkung von humanem MIA (Hemmung der Zellwanderung mit und ohne MIA in %).
B16 + mMIA: Test mit murinem MIA.
- Fig. 2: Inhibition von T-Cell-vermittelter cytotoxischer Aktivität durch MIA als %Lyse von CD4⁺ T-Zellen.
- Fig. 3: Inhibition der cytotoxischen Aktivität von LAK-Zellen durch MIA.
- Fig. 4: Inhibition der Phytohämagglutinin-abhängigen Lymphozytenproliferation durch MIA (MIA-Konzentration in ng/ml).
- Fig. 5: Inhibition der IL-2-stimulierten PBMC Proliferation durch MIA (MIA-Konzentration in ng/ml).
- Fig. 6: Plasmidkarte des Expressionsplasmids pQE40-MIA (Beispiel 5a).
- Fig. 7: Plasmidkarte des Vektors pCMX-PL1 (Beispiel 7a).

### Beispiel 1

### Isolierung des Melanom-inhibierenden Proteins aus HTZ 19-dM-Zellen.

HTZ 19-dM-Zellen werden als Monolayer in definiertem serumfreien Gewebekulturmedium (50 % Dulbecco's minimal essential medium, 50 % F-12, Boehringer Mannheim GmbH) mit 0,8 mmol/l L-Glutamin (Gibco, U.K.), nichtessentiellen Aminosäuren (Gibco, U.K.), 10 µg/ml Transferrin (Boehringer Mannheim GmbH, Katalog-Nr. 1073974), 30 nmol/l Natriumselenit (Sigma) und 4 pg/ml Gentamycin (Merck) kultiviert. Der Zellkulturüberstand dieser Kultur wird im Abstand von jeweils 3 - 4 Tagen abgenommen und bis zur Aufreinigung bei -70°C gelagert.

Zur Aufreinigung werden die Zellkulturüberstände durch 0,45 µm Filter (Becton Dickinson, Heidelberg) filtiert und durch Membran-Ultrafiltration mit Amicon YM 2-Membranen (Auschlußgrenze 2000 D, Amico Danvers Massachusetts, USA) auf ein Endvolumen von 1 % des Ausgangsvolumens aufkonzentriert. Das erhaltene Material wird für 30 Stunden gegen 0,1 mol/l Essigsäure dialysiert (Dialysemembran mit Ausschlußgrenze von 1000 D, Reichelt, Heidelberg) und anschließend bei 100 000 g für eine Stunde bei 4°C ultrazentrifugiert. Das Pellet wird verworfen und der Überstand zur weiteren Verarbeitung lyophilisiert.

Die lyophilisierten Dialysate werden in 1 mol/l Essigsäure aufgenommen und durch Gelpermeationschromatographie an einer Biogel P-10-Säule (Pharmacia, Uppsala, 2,6 x 100 cm; Bioge P⁻¹⁰, 200 - 400 Mesh, Biorad Laboratories, Richmond, California, USA) weiter aufgereinigt. Das Gelmaterial wird mit 1 mol/l Essigsäure bei 22°C äquilibriert und die Dialysate in einer Konzentration von 130 - 145 mg in 5 ml in 1 mol/l Essigsäure aufgetragen. Eluiert wird mit 1 mol/l Essigsäure bei einer Flußrate von 12 ml/Std. und das Eluat in 4 ml-Fraktionen aufgefangen. Über Bestimmung der Anti-Tumor-Aktivität (vgl. Beispiel 5) werden drei aktive Fraktionspools definiert, von denen der mittlere Pool, der einem Molekulargewicht von 8000 - 17000 D entspricht, über reverse phase HPLC weiter aufgereinigt wird. Dazu werden diese Fraktionen zunächst einmal lyophilisiert und dann in 0,1 % Trifluoressigsäure (TFA) aufgenommen. Jeweils 100 µl Aliquots der erhaltenen Lösung werden auf die reverse phase HPLC aufgetragen (Flußrate 0,5 ml/min). Es werden Fraktionen mit je 750 µl gesammelt. Weitere Daten der HPLC-Trennung:
- Gradientenprogramm:: Lösung A: 0,06 % TFA in Wasser
Lösung B: 0,056 % TFA, 80 % Acetonitril

2 - 25 % Lösung B in 5 min
25 - 50 % Lösung B in 120 min
50 - 100 % Lösung B in 5 min
zurück auf 2 % in 5 min
Säule: minoRPC (Pharmacia)
HPLC-Gradientenmischer, Pumpe und Detektor: Pharmacia

Das Eluat wird in 1,5 ml-Fraktionen aufgefangen. Aliquots werden lyophilisiert und, wie in Beispiel 5 beschrieben, auf Anti-Tumor-Wirkung hin untersucht.

Auf diese Weise können ca. 1 µg Melanom-inhibierendes Protein aus 5 1 Kulturüberstand gewonnen werden.

### Beispiel 2

### Beispiel 2a

### Klonierung der für das humane Melanom-inhibierende Protein codierenden cDNA

Nach Asp-N und Trypsin-Verdau des gereinigten Proteins und erneuter Aufreinigung der so erhaltenen Peptidfragmente werden die MIA-Aminosäuresequenzen im Sequenzierer bestimmt. Die C-terminale und eine nahe dem N-Terminus gelegene Peptidsequenz wurden als Grundlage zur Synthese zweier Primer ausgewählt. Bei den Primern handelt es sich um degenerierte Oligonukleotide mit angefügten Restriktionsenzymschnittstellen.

Der Querstrich (/) bedeutet, daß entweder die Base vor oder hinter dem Querstrich an dieser Stelle steht. Dieses Oligonukleotid ist eine Mischung aus 32 verschiedenen Molekülen, wodurch fast alle möglichen Codons abgedeckt sind. Lediglich an den Positionen 12 und 13 sind G-T Mismatches mit der Zielsequenz möglich, was die Stabilität der Hybride nicht erhöht, aber auch nicht vermindert. Am 5'-Ende hängt zusätzlich ein EcoRI-Linker, um ein eventuelles Produkt nach PCR leicht umklonieren zu können. Davor liegen am 5'-Ende noch 3 unspezifische Basen, damit die Restriktionsschnittstelle nicht ganz am Rand liegt, da Restriktionsenzyme dort weniger gut schneiden.

DP 1 entspricht den 8 C-terminalen Aminosäuren, ist 8-fach degeneriert und enthält eine Sal I Schnittstelle.

Für die spezifische Erststrangsynthese wurde der Primer DP 1 in dem folgenden Ansatz verwendet:
5 µl 10 x PCR Puffer
3 µl HTZ-19 Gesamt-RNA
wurden gemischt und 5 min auf 65°C erhitzt.

Dazu wurden pipettiert:
1 µl 100 mM MgCl₂
10 µl 2,5 mM dNTP
0,5 µl Placenta RNase Inhibitor
2 µl DP 1 (1 µg)
1 µl Reverse Transkriptase

Nach 1 h Inkubation bei 37°C wurde zu obigem Ansatz folgendes zugegeben:
1 µl UP 1 (1 µg)
5 µl 10 x PCR Puffer
70,5 µl H₂O
1 µl Taq-Polymerase

Die Amplifikation erfolgte 30 Zyklen mit dem folgenden Profil:
94°C 30 sec.
55°C 30 sec.
72°C 60 sec.

Nach dem letzten Zyklus wurde der Ansatz zur vollständigen Verlängerung aller Produkte noch 7 min. bei 72°C inkubiert.

Nach einer Phenol/Chloroform-Extraktion und EtOH-Fällung erfolgte der Verdau des PCR-Ansatzes mit EcoRI und SalI 2 h bei 37°C. Nach Enzymaktivierung folgte die Auftrennung in einem 5 % PAA-Gel und Elution des 320 bp großen Fragmentes über Nacht. Die Hälfte der Eluats wurde über Nacht mit 100 ng EcoRI/SalI verdautem pbluescript ligiert. Von den am nächsten Tag transformierten und auf SOB-Agar-Platten mit Amp und X-Gal/IPTG ausplattierten Bakterien (E. coli DH5a) konnte eine rekombinante weiße Kolonie gepickt werden.

Nach Isolation des Plasmids erfolgte die Sequenzierung des umklonierten Inserts mit dem T-7 Deaza Sequencing-Kit von Pharmacia. Als Primer standen der T-3 und T-7 Primer (Stratagene) zur Verfügung. Aus der Überlappung der abgelesenen Sequenzen ergab sich folgendes Bild (Primer fett gedruckt):

Für die Klonierung der vollständigen cDNA stand eine Lambda gtll cDNA-Bibliothek zur Verfügung, die aus der RNA von in definiertem Medium (dM) wachsenden HTZ-19 Melanomzellen synthetisiert worden war.

Insgesamt wurden 25 Platten mit je 8000 pfu ausplattiert, je 2 Nitrocellulose-Filter aufgelegt und mit dem durch Nick-Translation markierten gereinigten MIA-PCR Insert hybridisiert (50 ml Hybridisierungslösung, 2 x 10⁶ cpm/ml). Nach zweitägiger Autoradiographie konnten mehrere Signale erhalten werden, die auf beiden Filtern ein übereinstimmendes Hybridisierungssignal ergaben. Die zugehörigen Phagen-Plaques wurden isoliert und einer Nachuntersuchung unterzogen. Dabei wurden von jedem isolierten Plaque 4 Verdünnungsstufen ausplattiert und die Platten mit 100-300 pfu für zwei weitere Hybridisierungen verwendet.

Aus den so isolierten Plaques konnte nach einer 50 ml Übernacht-Kultur die Lambda-DNA isoliert werden, 40 µg mit EcoRI verdaut und in einem 5% PAA-Gel aufgetrennt werden. Das Insert wurde eluiert und 8 ng für die Ligation mit 100 ng EcoRI verdautem, dephosphoryliertem Vektor (pbluescript, Stratagene) eingesetzt. Die Hälfte der Ligationsansätze wurden für die Transformation kompetenter E. coli DH5a eingesetzt, die auf SOB/Amp-Platten mit IPTG und X-Gal für die Blau/Weiß Selektion ausplattiert wurden. Die rekombinanten Kolonien wurden abgeimpft, die Plasmid-DNA isoliert und die Inserts sequenziert. Die Sequenz des Inserts mit der vollständigen codierenden Sequenz ist unter SEQ ID NO 1 gezeigt.

Ein auf diese Weise erhaltenes Plasmid ist pbs L7MIA, welches bei der Deutschen Sammlung von Mikroorganismen und Zellkulturen GmbH (DSM) in Braunschweig, Deutschland am 14.07.93 hinterlegt wurde (DSM 8420).

Alle für die Klonierung verwendeten Methoden sind ausführlich in J. Sambrook, E.F. Fritsch, T. Maniatis (1989), Molecular cloning: a laboratory manual, 2nd edition, Cold Spring Harbor Laboratory Press, Cold Spring Harbor, USA, beschrieben.

### Beispiel 2b

### Klonierung des für das humane Melanom-inhibierende Protein codierenden Gens

Eine humane genomische DNA Bibliothek im Bacteriophagen Lambda FIX II (Elgin et al. (1991), Strategies 4, 8-9), kommerziell erhältlich von Stratagene (Heidelberg), wurde nach etablierten Methoden (Sambrook et al. Molecular Cloning, Cold Spring Harbor Laboratory, 1989) auf Nitrocellulose-Filter plattiert. Zur Verwendung als Hybridisierungs-Probe wurde die für humanes MIA codierende cDNA aus Beispiel 2a radioaktiv markiert und entsprechend etablierten Techniken verwendet (Sambrook et al. (1989), Molecular Cloning, Cold Spring Harbor Laboratory Press). Vorhybridisierung (2 Stunden) und Hybridisierung (16 Stunden) erfolgten bei 60°C in 6 x SSC, 5 x Denhardt's Lösung, 100 µg ml Lachssperma-DNA und 0.1% SDS. Die ³²P-dCTP-markierte Probe wurde dem Hybridisierungsansatz in einer Konzentration von 1 x 10⁶ cpm/ml zugesetzt. Anschließend wurden die Filter bei 60°C zweimal 20 Minuten gewaschen in 2 x SSC, 0.1 % SDS, dann zweimal 20 Minuten in 1 x SSC, 0.1 % SDS und schließlich zweimal 20 Minuten in 0.25 x SSC, 0.1 % SDS. Die Filter wurden daraufhin getrocknet und 24 bis 48 Stunden auf Roentgenfilm exponiert. Die bei diesem Verfahren ein positives Hybridisierungssignal liefernden Plaques wurden isoliert und durch Rehybridisierung bestätigt. Die in diesen Phagen als Insert enthaltene humane genomische DNA wurde durch Southern-Hybridisierung unter Verwendung von MIA cDNA-Proben charakterisiert. Hierzu wurde die Phagen DNA mit der Restriktionsendonuklease XbaI gespalten, auf einem 0.8 % Agarose-Gel aufgetrennt und anschließend nach Southern (J.Mol.Biol. 98 (1975), 503) auf Nitrocellulose transferiert. Die so erhaltenen Filter wurden unter den oben beschriebenen Bedingungen mit der kompletten MIA cDNA als Probe hybridisiert, wobei zwei XbaI Fragmente von ca. 1.4 kb und ca. 2.2 kb positive Signale lieferten. Diese beiden Fragmente wurden jeweils in das zur Sequenzierung geeignete und bei Stratagene (Heidelberg) käuflich erhältliche Plasmid pBluescriptSK- (Short et al. (1988), Nucl. Acids res. 16, 7583 - 7600; Alting-Meese und Short (1989), Nucl. Acids res. 17, 9494) kloniert. Die gesamte, für das humane MIA codierende Sequenz ist in vier Exons lokalisiert, die in SEQ ID NO:3 mit ihren flankierenden Sequenzen gezeigt sind. Da nicht untersucht wurde, ob zwischen den beiden XbaI-Fragmenten, auf denen die MIA Exons lokalisiert sind, sich eines oder sogar mehrere weitere XbaI-Fragmente befinden, kann nicht ausgeschlossen werden, daß Intron 2 in Wirklichkeit wesentlich größer ist.

### Beispiel 2c

### Klonierung der für das murine Melanom-inhibierende Protein codierenden cDNA

Eine kommerziell erhältliche (Novagene, New York) cDNA-Bibliothek aus einem 13.5 Tage alten Maus-Embryo im Vektor Lambda EXlox (Palazzolo et al. (1990), Gene 88, 25 - 36) wurde wie in Beispiel 2a beschrieben plattiert. Als Hybridisierungsprobe wurde die für humanes MIA codierende cDNA aus Beispiel 2a in radioaktiv markierter Form eingesetzt. Die Hybridisierungsbedingungen waren identisch mit den in Beispiel 2b beschriebenen, mit Ausnahme der Temperaturen bei Hybridisierung und Waschung, die hier 55°C betrugen. Die in den so erhaltenen und durch Rehybridisierung bestätigten Plaques vorliegenden cDNA-Inserts wurden sequenziert. Die Sequenz des Inserts mit der vollständigen codierenden DNA des murinen MIA ist in SEQ ID NO 4 gezeigt.

### Beispiel 2d

### Klonierung des für das murine Melanom-inhibierende Protein codierenden Gens

In diesem Fall wurde eine murine genomische DNA-Bibliothek (aus der Leber einer adulten BALB/C Maus im Vektor EMBL3 (Frischauf et al. (1983), J.Mol.Biol. 170, 827), käuflich erhältlich bei Clontech, Palo Alto CA) in analoger Weise wie in Beispiel 2b beschrieben unter Verwendung der murinen MIA cDNA aus Beispiel 2c als Probe abgesucht. Die Bedingungen waren identisch zu den in Beispiel 2b beschriebenen. Auch das weitere Vorgehen erfolgte in analoger Weise.

### Beispiel 3

### Beispiel 3 a

### Bestimmung der antiproliferativen Wirkung des Melanom-inhibierenden Proteins auf Tumorzellen

Zur Bestimmung der antiproliferativen Wirkung des Melanom-inhibierenden Proteins bzw. der gemäß Beispiel 1 erhaltenen Proteinfraktionen auf Melanomzellen werden exponentiell wachsende HTZ 19-dM-Zellen für 24 Stunden in 96er Mikrokulturplatten (Costar, Zürich) in jeweils 100 µl serumfreiem Medium (s. Beispiel 1) mit einer Dichte von 3 x 10³-Zellen pro Vertiefung ausgesät (gemäß Chambard et al., J. Cell. Physiol. 135 (1988), 101 - 107). Die Zellen werden dann mit der zu untersuchenden Proteinfraktion für 4 - 5 Tage bei 37°C/5 % CO₂ inkubiert. Nach Zugabe von jeweils 1 µCi ³H Thymidin (spezifische Aktivität 23 Ci/mmol, Amersham Buchler, Braunschweig, Deutschland) werden die Zellen 8 Stunden unter identischen Bedingungen weiter inkubiert und anschließend der ³H Thymidin-Einbau in die zelluläre DNA nach Säurefällung in üblicher Weise über einen Flüssigszintillationszähler gemessen. Die Aktivität der untersuchten Proteinfraktion wird als Prozentsatz des ³H Thymidin-Einbaus dieser behandelten Zellen, gegenüber dem ³H Thymidin-Einbau in unbehandelten Kontrollzellen ausgedrückt. Unter Verwendung verschiedener Konzentrationen des isolierten Melanom-inhibierenden Proteins kann eine Konzentration ermittelt werden, bei welcher dieser ³H Thymidin-Einbau um 50 %, gegenüber der unbehandelten Kontrolle, gehemmt wird (IC 50-Wert in der folgenden Tabelle 1).

**Tabelle 1**

| Antiproliferative Wirkung des Melanom-inhibierenden Proteins auf verschiedene Tumorzellen | |
|---|---|
| Tumorzellinie | IC 50 (µg/ml)¹⁾ |
| a) Melanomzellinien | |
| HTZ 19-dM | 1,2 |
| ATCC HTB 69 1 | 3,7 |
| HTZ 320 | 1,35 |
| HTZ 318 | 3,5 |
| ATCC CRL 1424 | 2,1 |

| b) Neuroblastom- | |
|---|---|
| linien | |
| Kelly 1 | 80 |

| c) Glioblastom | |
|---|---|
| ATCC HTB17 | 10 |

| d) Astrocytom | |
|---|---|
| HTZ 243 | 5 |
| HTZ 209 | 5 |

| | |
|---|---|
| ¹⁾ Verwendet wurde das Protein nach dem ersten Reinigungsschritt (nach BiogelP10 Säule, Faktor 50-100 geringere Aktivität als nach vollständiger Reinigung) | |

### Beispiel 3b

### Bestimmung der invasionshemmenden Wirkung des Melanom-inhibierenden Proteins auf Tumorzellen

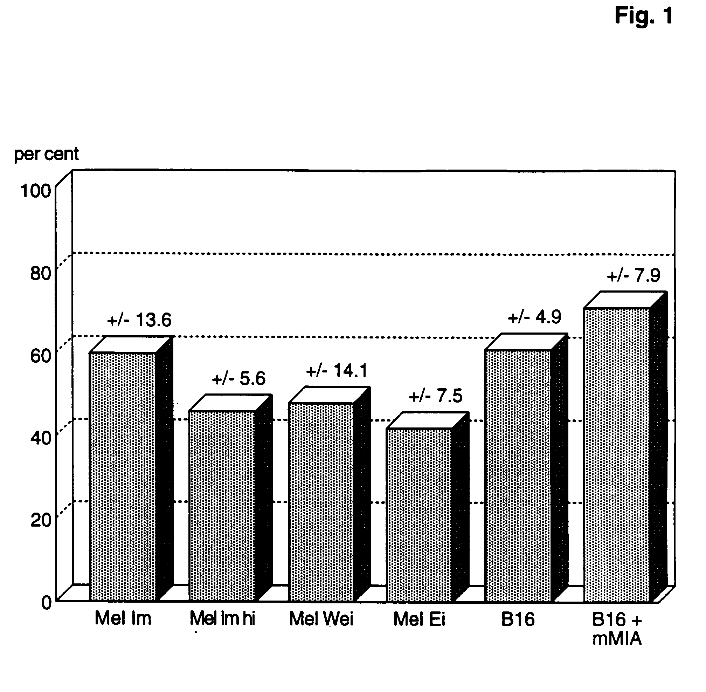

Zur Bestimmung der invasionshemmenden Wirkung von MIA wird ein modifiziertes Boyden-Kammer-System (Albini et al. (1987), Cancer Res. 47, 3239 - 3245) verwendet. Die Kammern wurden von der Firma Costar (Blind Well Chamber No. 441200) bezogen. Zur Simulierung einer Basalmembran-ähnlichen Barriere zwischen dem Chemoattraktans in der unteren Kammer und den Zellen in der oberen Kammer werden auf den Polycarbonatfilter (Porengröße 8 µm, Costar No. 150446) 52 µl Matrigel (Becton Dickinson Cat. No. 40234) aufgebracht. Als Chemoattraktans wird die untere Kammer mit 210 µl Fibroblasten-konditioniertem Medium befüllt. Dieses wird wie folgt gewonnen: Fibroblasten aus normaler menschlicher Haut werden zwischen der 10. und 20. Passage für 24 Stunden in DMEM-Medium (Gibco) ohne Zusatz von foetalem Kälberserum gehalten. Das so konditionierte Medium wird unverdünnt als Chemoattraktans eingesetzt. In die obere Kammer der Boyden-Apparatur werden 2 x 10⁵ der zu untersuchenden Tumorzellen in 800 µl DMEM (Gibco, ohne foetales Kälberserum) mit, bzw. ohne MIA-Wirkstoff gegeben. Humane Tumorzellen (siehe Beispiel 3a oder Beispiel 8) oder tierische Tumorzellen wie etwa B16 (ATCC CRL 6322) können mit der beschriebenen Methode auf Inhibierung ihres Migrationsverhaltens durch MIA getestest werden. Ohne Zugabe des Melanom inhibierenden Proteins wandern ca 10 % Tumorzellen innerhalb von ca 4 Stunden aus der oberen Kammer in die untere Kammer, wo sie an der Unterseite der Matrigel-Membran haften bleiben. Sie werden dort fixiert, gefärbt und anschließend gezählt. Wird der oberen Kammer das humane oder murine Melanom inhibierende Protein MIA zugesetzt, so wird die Wanderung der Zellen stark inhibiert. Fig. 1 zeigt die erhaltenen Hemmwerte ([Zellzahl in der unteren Kammer, Experiment mit MIA] / [Zellzahl in der unteren Kammer, Experiment ohne MIA] x 100 %).

### Beispiel 4

### Bestimmung der immunologischen Aktivität

### Beispiel 4a

### MIA hemmt T-Zell vermittelte zytotoxische Aktivität

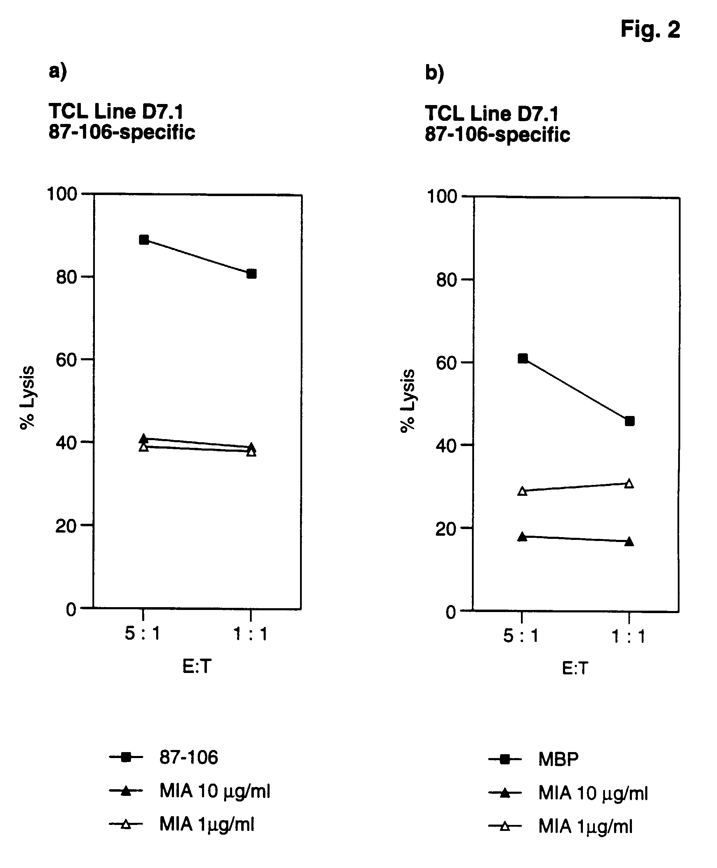

Die gegen ein Peptid aus myelin basic protein (MBP, Aminosäureposition 87 - 106) spezifische CD4⁺ T-Zellinie D7.1 ist in der Lage, in einem Standard ⁵¹Cr-Release Assay sowohl MBP- als auch Peptid 87-106 präsentierende Targets zu lysieren (Targets: Daudi-Zellen, R. Martin, U. Utz, J.E. Coligan, J.R. Richert, M. Flerlage, E. Robinson, R. Stone, W.E. Biddison, D.E. McFarlin, H.F. MacFarland, Diversity in fine specificity and T cell receptor usage of the human CD⁴⁺ cytotoxic T-cell response specific for the immunodominant myelin basic protein peptide 87 - 106, J. Immunol. (1992), 148 (5), 1359 - 1366). Nach Zugabe von MIA (eingesetzte Menge entspricht ca. 50 - 100 ng/ml gereinigten MIA) wird die Peptid-spezifische Zytotoxizität um ca. 55 % (Fig. 2 a), die MBP-spezifische Zytotoxizität um ca. 50 % (Fig. 2 b) gehemmt. Die Hemmung zeigt erwartungsgemäß eine geringe Abhängigkeit vom Effektor-Target-Verhältnis (E:T), das hier mit 1:1 bzw. 5:1 sehr niedrig angesetzt wurde und damit hochspezifisch ist (Fig. 2).

### Beispiel 4b

### MIA hemmt die zytotoxische Aktivität von Lymphokinaktivierten peripheren Blutlymphozyten (LAK-Zellen)

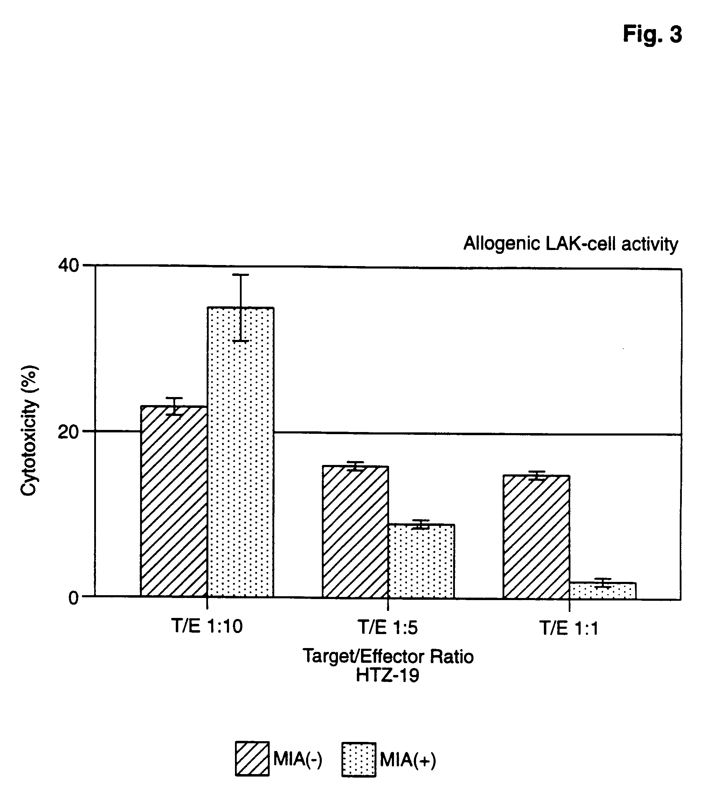

LAK-Zellen sind nicht klonal expandierte, Lymphokin-aktivierte periphere Blutlymphozyten, vorwiegend T-Lymphozyten (A.A. Rayner, E.A. Grimm, M.T. Lotze, E.W. Chu, S.A. Rosenberg, Lymphokine activated killer (LAK) cells: Analysis of factors relevant for immunotherapy of human cancer, Cancer 55 (1985), 1327 - 1333). Sie werden in vielen immunologischen Therapie-Ansätzen der Tumortherapie standardisiert eingesetzt. In dem hier gezeigten Experiment werden LAK-Zellen auf ihre Zytotoxizität gegenüber HTZ-19 Melanom-Zellen als Targets in einem Microzytotoxizitäts-Assay geprüft. In Effektor-Target-Verhältnissen von 1:1, 5:1 und 10:1 kommt es zu einer maximalen Zytotoxizität (CTX) von knapp 40 %. Diese wird nach Zugabe von MIA (Konzentration wie in Beispiel 4 a) stark gehemmt, maximal um 80 % bei niedrigem Effektor-Target-Verhältnis, wie es am ehesten lokal - am Tumor selbst - zu erwarten ist (Fig. 3).

### Beispiel 4c

### MIA hemmt die IL-2 abhängige bzw. Phytohämagglutininabhängige Lymphozytenproliferation

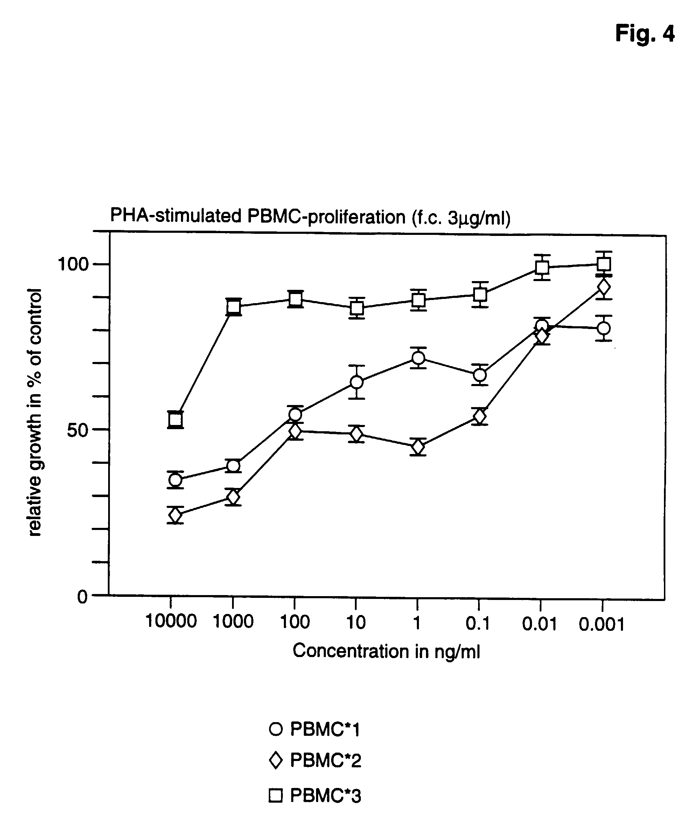

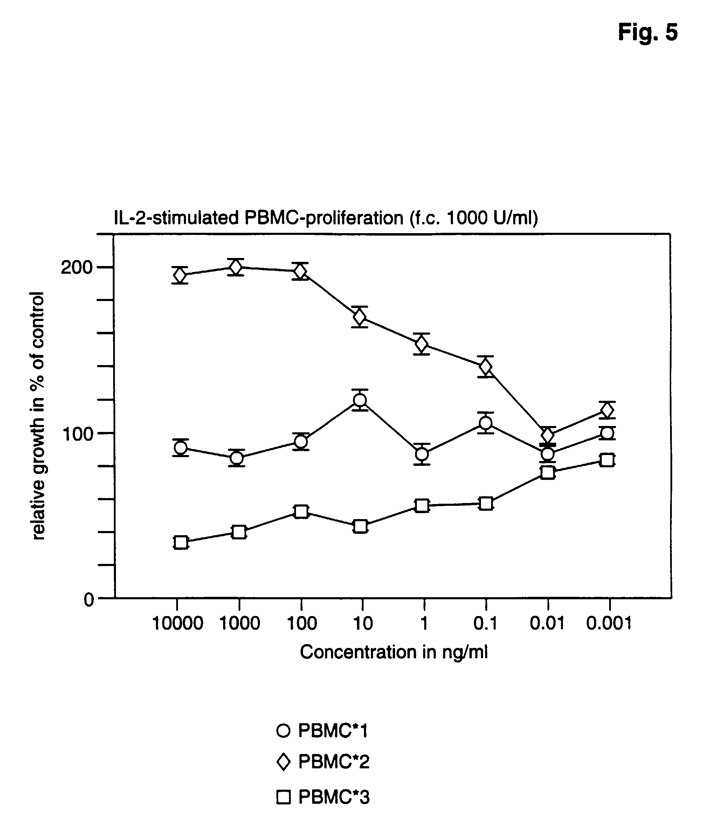

Periphere Blutlymphozyten (PBMC) lassen sich klassischerweise mit Phythämagglutinin (PHA) und Interleukin-2 (IL-2) standardisiert stimulieren (J.E. Coligan, A.M. Kruisbeek, D.H. Margulies, E.M. Shevach, W. Strober, Current protocols in immunology, NIH Monograph, J. Wiley Sons, New York, 1992). Hierbei werden mit PHA fast ausschließlich T-Zellen stimuliert, mit IL-2 vorwiegend T-Lymphozyten, aber auch B-Zellen mit IL-2-Rezeptor. Unter Co-Inkubation mit MIA läßt sich im angegebenen Dosierbereich (Proteineinheit: nach dem ersten Reinigungsschritt (Biogel P10-Säule), Aktivität ist um den Faktor 50 - 100 geringer als nach vollständiger Reinigung) eine sehr starke Hemmung der PHA-Antwort erreichen (Fig. 4). Die IL-2-Antwort wird im höheren Dosisbereicht gehemmt (Fig. 5).

### Beispiel 5

### Rekombinante Expression von MIA als Fusionsprotein in E.coli

### Beispiel 5a

### Konstruktion von Expressionsvektoren

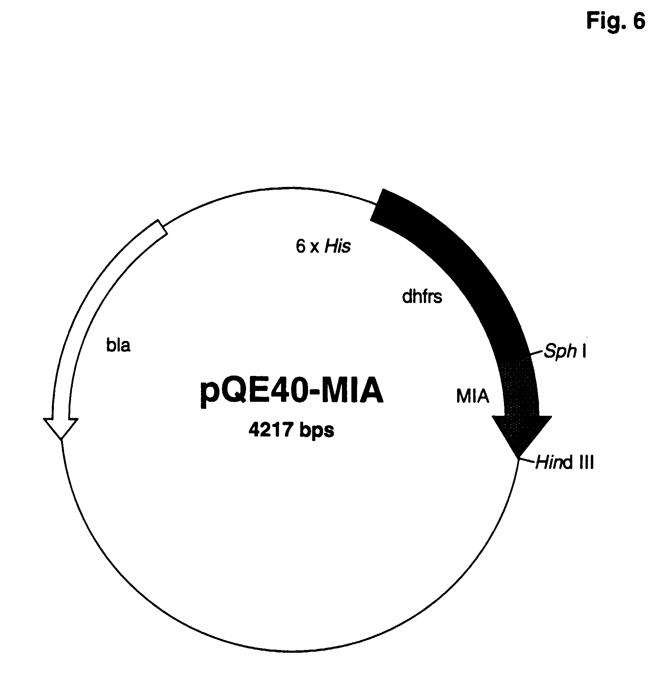

Zur Expression von MIA als Fusionsprotein mit einem als "Carrier" in E.coli geeigneten Protein kann beispielsweise der käuflich erhältliche Vektor pQE40 (Cat. No. 33403, DIAGEN GmbH, Düsseldorf) verwendet werden. In diesen Vektor wird zwischen die je einmal vorhandenen Restriktionsschnittstellen SphI und HindIII ein für die reife Form von MIA codierendes cDNA-Fragment insertiert. Ein derartiges Fragment wird am einfachsten durch PCR-Amplifikation nach bekannten Techniken hergestellt unter Verwendung der klonierten MIA cDNA als Matrize und zweier geeigneter Primer (5'-GATGCATGCGGTCCTATGCCCAAGCTG-3' (SEQ ID NO:9) und 5'-GATAAGCTTTCACTGGCAGTAGAAATC-3' (SEQ ID NO:10)). Das erhaltene PCR-Fragment wird mit SphI und HindIII geschnitten und in den ebenso behandelten Vektor pQE40 ligiert. Das resultierende Plasmid exprimiert ein Fusionsprotein aus DHFR (Dihydrofolat-Reduktase als "Carrier") und MIA. Um aus diesem Fusionsprotein freies MIA proteolytisch abspalten zu können, wird ein DNA-Segment zwischen DHFR und MIA einkloniert, welches für die Erkennungssequenz der IgA-Protease (Ser Arg Pro Pro/Ser) codiert. Dies geschieht durch Öffnen des Expressionsplasmides mit BglII (Partialrestriktion mit anschließender Isolierung des linearisierten Vektors) und SphI und anschließende Insertion eines Adaptors (5'-GATCTAGCCGGCCGCCCAGCCCGGCATG-3' (SEQ ID NO:11) und 5'-CCGGGCTGGGCGGCCGGCTA-3' (SEQ ID NO:12) hybridisiert zum Doppelstrang). Das resultierende Expressionsplasmid pQE40-MIA codiert für ein Fusionsprotein aus DHFR und MIA, wobei sich zwischen DHFR und MIA eine Spaltstelle für IgA-Protease befindet. Das Fusionsprotein trägt am N-Terminus 6 Histidine, die den Angaben des Herstellers zufolge zur Aufreinigung des Fusionsproteins mit Hilfe von Ni-Chelat-Gelmaterialien verwendet werden können. Derartige Verfahren sind in EP-A 0 282 042 und EP-A 0 253 303, deren Inhalt Gegenstand der Offenbarung dieser Anmeldung ist, beschrieben. Fig. 6 zeigt das Expressionsplasmid pQE40-MIA.

Der Gehalt von MIA läßt sich optimieren, indem das Carrier-Protein DHFR eliminiert und durch ein möglichst kleines Peptid ersetzt wird, welches dieselben Funktionen erfüllt. Derartige geeignete Peptide sind beispielsweise MetArgGlySerHisHisHisHisHisHisGlySerSerArgProPro (SEQ ID NO:13) (dieses Peptid kann durch IgA-Protease von der unmittelbar darauf folgenden Aminosäure-Sequenz des reifen MIA abgespalten werden; derartige Verfahren sind in der WO 91/11520 beschrieben, deren Inhalt Gegenstand der Offenbarung dieser Anmeldung ist) oder MetArgGlySerHisBisBisHisHisHisGlySerValAspAspAspAspLys- (SEQ ID NO:14) (dieses Peptid kann durch Enterokinase von der unmittelbar darauf folgenden Aminosäure-Sequenz des reifen MIA abgespalten werden). Expressionsplasmide, die für derartige MIA-Peptidfusionen codieren, können wie folgt hergestellt werden: Eine PCR-Amplifikation mit MIA cDNA (Seq.ID No 1) als Matrize und den Primern 5'-AAAAAGGATCCAGCCGGCCGCCCGGTCCTATGCCCAAGCTGGC-3' (SEQ ID NO:15) und 5'-GGCGAGCAGCCAGATCTCCATAG-3' (SEQ ID NO:16) liefert ein Fragment, welches mit BamHI und BglII nachgeschnitten wird. Der Expressionsvektor pQE40-MIA wird ebenfalls mit BamHI und BglII restringiert, das kleinere der entstehenden Fragmente wird verworfen und durch das beschriebene PCR-Fragment ersetzt. Hierdurch wird ein Expressionsvektor erhalten, der nach Induktion das Fusionsprotein MetArgGlyserHisHisHisBisBisBisGlySerSerArgProPro-MIA (SEQ ID NO:13) exprimiert. Das Fusionsprotein MetArgGlySerHisHisHisHisHisHisGlySerValAspAspAspAspLys-MIA (SEQ ID NO:14) wird in völlig analoger Weise unter Verwendung der Primer 5'-AAAAAAGGATCCGTTGATGATGACGATAAAGGTCCTATGCCCAAGCTGGC-3' (SEQ ID NO:17) und 5'-GGCGAGCAGCCAGATCTCCATAG-3' (SEQ ID NO:16) erhalten.

Weitere, ähnliche Fusionsproteine aus Peptiden und MIA können in analoger Weise hergestellt und unter der Kontrolle geeigneter (vorzugsweise starker und induzierbarer) Promotoren in eines der zahlreichen für E.coli beschriebenen Plasmide einkloniert und zur Expression gebracht werden. Eine weitere Alternative stellt die Fusion von MIA mit einem Peptid dar, welches in E.coli zu Sekretion des Fusionsproteines ins Periplasma mit nachfolgender Abspaltung und Freisetzung von MIA führt. Dieses Verfahren ist in WO 88/09373 beschrieben, deren Inhalt Gegenstand der Offenbarung dieser Anmeldung ist.

### Beispiel 5b

### Expression von Fusionsproteinen und Gewinnung von MIA

Das Expressionsplasmid pQE40-MIA (oder ein ähnliches Expressionsplasmid, welches beispielsweise durch Verwendung eines anderen Grundvektors oder eines anderen "Carrier"-Proteins oder -Peptides erhalten werden kann; derartige Alternativen sind außer in Beispiel 5a auch beschrieben in Methods of Enzymology 185 (Gene Expression Technology), Hrsg. David V. Goeddel, Academic Press 1991) wird in einen geeigneten E.coli Stamm transfiziert, der über eine hinreichende Expression des lac-Repressors verfügt, so daß eine induzierbare Expression des MIA-Fusionsproteins erzielt werden kann. Geeignet hierfür ist beispielsweise der Stamm E.coli M15[pREP4], der zusammen mit pQE40 käuflich erhältlich ist (Diagen GmbH, Düsseldorf), oder aber andere E.coli-Stämme wie etwa E.coli UT5600 (Earhart et al. (1979), FEMS Microbiology Letters 6, 277-280) oder E.coli BL21 (Grodberg und Dunn (1988), J.Bacteriol. 170, 1245-1253), die zuvor mit einem lac-Repressor exprimierenden Helfer-Plasmid wie etwa pUBS520 (Brinckmann et al. (1989), Gene 85, 109-114) oder pUBS500 (EP-B 0 373 365) transfiziert wurden. MIA kann dann wie folgt gewonnen werden: E.coli M15 [pREP4/pQE40-MIA] wird auf LB-Medium bis zu einer optischen Dichte von 0.6 (gemessen bei 550 nm) kultiviert, dann IPTG (Isopropyl-β-D-thiogalactopyranosid, Boehringer Mannheim GmbH) in einer Endkonzentration von 1 mM zugegeben und anschließend 4 Stunden weiterkultiviert. Die Zellen werden abzentrifugiert, in 100 mM Natrium-Phosphat-Puffer pH 7.5 mit 300 mM NaCl aufgenommen und durch dreimaliges Einfrieren und Auftauen mit anschließender Ultraschallbehandlung lysiert. Zu dem durch Zentrifugation geklärten Lysat wird Ni-NTA-Agarose (Diagen GmbH) unter Berücksichtigung der vom Hersteller angegebenen maximalen Bindekapazität zugegeben und über Nacht bei Raumtemperatur unter Mischen inkubiert. Das so mit Fusionsprotein beladene Gelmaterial wird niedertourig abzentrifugiert, zweimal mit 100 mM Natrium-Phosphat-Puffer pH 7.5 und zweimal mit Natrium-Phosphat-Puffer pH 6.1 gewaschen. Anschließend wird MIA aus dem Fusionsprotein abgespalten durch Inkubation des Gelmateriales mit IgA-Protease (Boehringer Mannheim GmbH) in 100 mM Natrium-Phosphat-Puffer pH 7.5 über Nacht bei 37°C. Das Gelmaterial wird durch Zentrifugation abgetrennt und der MIA-haltige Überstand nach Sterilfiltration in den in den Beispielen 3 und 4 beschriebenen Aktivitätstests eingesetzt.

### Beispiel 6

### Rekombinante Expression von fusionsfreiem MIA in E.coli

Die für MIA codierende DNA-Sequenz wird in einer Weise modifiziert, die eine effiziente Expression in E.coli ermöglicht. Zu diesem Zweck wird eine PCR-Amplifikation mit humaner MIA cDNA (Seq. ID No 1) als Matrize und den Primern 1 (5'-AAAAACATATGGGACCAATGCCAAAATTAGCAGATCGTAAATTATGTGCAGATCAGGAG-3' (SEQ ID NO:19) und 2 (5'-AAAAAAAGCTTTCACTGGCAGTAGAAATC-3' (SEQ ID NO:20)) durchgeführt. Primer 1 verändert die MIA codierende Sequenz in dem N-terminalen Bereich so, daß bei unveränderter MIA-Aminosäure-Sequenz die DNA- und damit die mRNA-Sequenz für E.coli optimiert wird. Das Startcodon Met wird hinzugefügt und an eben dieser Stelle eine Erkennungssequenz der Restriktionsendonuklease NdeI eingebracht, die eine anschließende Klonierung des so modifizierten MIA codierenden Fragmentes in einen Vektor gestattet, der einen (vorzugsweise starken und induzierbaren) Promoter und eine Translations-Inititations-Sequenz ("Shine-Dalgarno-Sequenz") mit nachfolgend plazierter NdeI-Schnittstelle enthält. Primer 2 dient als 3'-Gegenprimer und enthält eine HindIII-Schnittstelle, so daß die Insertion des modifizierten MIA codierenden Fragmentes in den Vektor als NdeI HindIII Fragment erfolgen kann. Die derart modifizierte MIA codierende Sequenz ist in SEQ ID NO:18 gezeigt. Ein derart hergestelltes Expressionsplasmid ist p11379, welches unter der Hinterlegungsnummer DSM 9267 am 29.06.94 bei der Deutschen Sammlung von Mikroorganismen und Zellkulturen GmbH, D-38124 Braunschweig, hinterlegt wurde.

Ein Expressionsplasmid für fusionsfreies MIA wird zur Expression in einen geeigneten E.coli Stamm transfiziert. Bei Verwendung eines durch lac-Repressor kontrollierten Expressionsplasmides wie pl1379 sind dies Stämme, die über eine hinreichend hohe intrazelluläre Konzentration an lac-Repressor verfügen. Solche Stämme können durch Transfektion eines zweiten Plasmides wie pREP4 (Diagen GmbH), pUBS500 oder pUBS520 (Brinckmann et al. (1989), Gene 85, 109-114) hergestellt werden. Die verwendeten E.coli-Stämme sollten vorzugsweise eine geringe zelleigene Protease-Aktivität aufweisen, wie dies beispielsweise bei E.coli UT5600 (Earhart et al. (1979), FEMS Microbiology Letters 6:277-280), bei E.coli BL21 (Grodberg und Dunn (1988), J.Bacteriol. 170:1245-1253) oder bei E.coli B der Fall ist. Die Expressionskultivierung erfolgt dann analog wie in Beispiel 5b beschrieben. Zur Gewinnung von MIA wird das als Proteinaggregat aus E. coli erhaltene MIA nach den in EP 241 022, EP 364 926, EP 219 874 und DE 40 37 196 beschriebenen Verfahren aufgearbeitet.

Im Detail wird hierzu beispielsweise wie folgt vorgegangen: MIA-haltige Proteinaggregate aus E.coli-Fermentationen (sogenannte "Inclusion Bodies") werden in 6 M Guanidinium-Hydrochlorid, 100 mM TrisHCl pH 8, 1 mM EDTA solubilisiert, anschließend auf pH 3-4 eingestellt und gegen 4 M Guanidinium-Hydrochlorid pH 3,5 dialysiert. Die Renaturierung des solubilisierten Proteins erfolgt dann in 1 M Arginin pH 8, 1 mM EDTA, 5 mM GSH (Glutathion reduziert) und 0,5 mM GSSG (Glutathion oxidiert). Aus dem Renaturierungsansatz kann MIA beispielsweise nach Zusatz von 1,4 M Ammoniumsulfat durch Adsorption an hydrophobe Gelmatrices wie Fractogel TSK Butyl (E. Merck, Darmstadt) und anschließende Elution in 20 mM TrisHCl pH 7 gewonnen werden.

### Beispiel 7

### Rekombinante Expression von MIA in eukaryontischen Zellen

### Beispiel 7a

### Rekombinante Expression von MIA in Säugerzellen

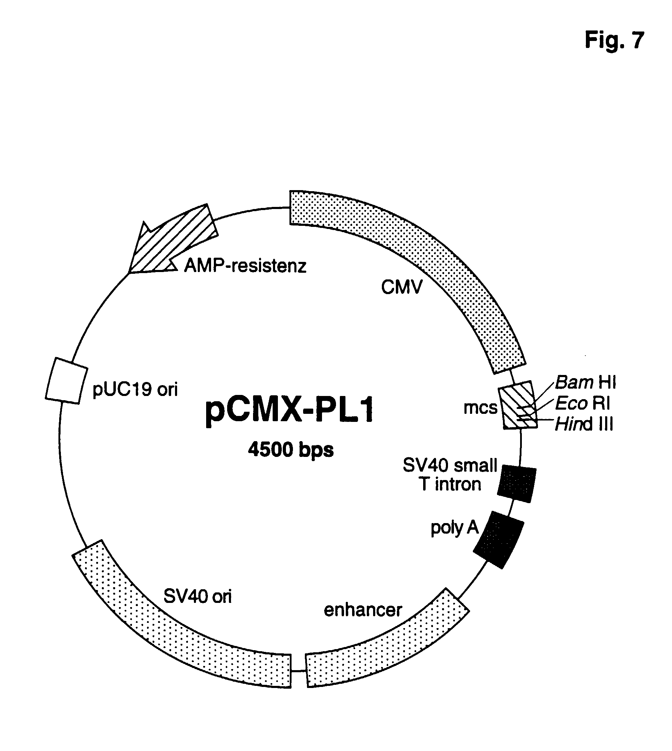

Hierzu werden die humane (SEQ ID NO:1) oder murine (SEQ ID NO:4) MIA cDNA oder die entsprechenden genomischen DNA-Segmente in einen Vektor ligiert, in welchem sie ausgehend von einem starken Promoter-Enhancer-System in Säugerzellen transkribiert werden (im Falle der genomischen MIA Fragmente ist dieser Schritt notwendig, da die MIA eigenen Promotoren nur in bestimmten Zelltypen, beispielsweise Melanomen, aktiv und daher nicht für eine allgemeine rekombinante Expression geeignet sind. Eine Expression kann jedoch auch wie in Beispiel 9 beschrieben durch homologe Rekombination in vitro erreicht werden. Derartige Promotoren und Enhancer stammen zumeist aus Viren wie SV40, hCMV, Polyoma oder Retroviren. Alternativ können auch Promoter-Enhancer-Systeme verwendet werden, die für einen bestimmten Zell- oder Gewebetyp spezifisch sind, wie etwa WAP-, MMTV- oder Immunglobulin-Promoter, oder aber solche Systeme, die induzierbar sind, wie etwa Metallothionein-Promoter. Ein solcher Vektor ergänzt die MIA-cDNA (falls eine solche verwendet wird) mit Donor- und Akzeptor-Signalen für die RNA-Prozessierung sowie einem Signal für poly-A-Addition. Ein solcher geeigneter Vektor ist beispielsweise pCMX-pL1 (Umesono et al. (1991), Cell 65, 1255-1266), der in Fig. 7 dargestellt ist. In die einzige EcoRI-Schnittstelle dieses Vektors wird die mit EcoRI-Linkern versehene MIA cDNA ligiert, wobei durch Restriktionsanalyse mit Hilfe der übrigen Schnittstellen im Polylinker dieses Vektors (siehe SEQ ID NO:23) sichergestellt wird, daß die Orientierung der MIA-cDNA in Ableserichtung des CMV-Promoters vorliegt. Völlig analog wird bei einer Klonierung in andere Vektoren vorgegangen, z.B. in pCDNA3 (Invitrogen, San Diego/USA) oder pSG5 (Stratagene, LaJolla/USA). Die DNA der so erhaltenen Expressionsplasmide wird aus E.coli präpariert und mit für den jeweiligen Zelltyp spezifischen Techniken in die Säugerzellen transfiziert (Methods of Enzymology 185 (Gene Expression Technology), Hrsg. David V. Goeddel, Academic Press 1991, Sektion V). Das Expressionsplasmid pCMX-pL1-MIA wird in die humane Teratocarcinoma-Linie PA-1sc9177 (Büttner et al. (1991), Mol. Cell. Biol. 11, 3573-3583) nach beschriebenen Methoden (Büttner et al. (1993), Mol. Cell. Biol. 13, 4174 - 4185) transfiziert, wobei 200 000 Zellen pro 100 mm Kulturschale mit 5 µg DNA transfiziert werden. Die Zellen werden nach der Transfektion in MEM (Gibco) ohne Zusatz von foetalem Kälberserum kultiviert, wobei nach 48 Stunden im Zellkulturüberstand MIA nachweisbar ist.

### Beispiel 7b

### Rekombinante Expression von MIA in Insektenzellen

Zur Expression in Insektenzellen wird ein für MIA codierendes DNA-Segment, vorzugsweise die humane MIA cDNA (SEQ ID NO 1), in Vektoren eingebracht, die sich von AcMNPV (Autographa californica nuclear polyhedrosis virus) oder BmNPV (Bombyx mori nuclear polyhedrosis virus) ableiten. Hierzu wird die MIA cDNA zunächst unter die Kontrolle eines für Insektenzellen geeigneten, starken Promoters gebracht (O'Reilly DR, Miller LK und Luckow VA 1992; Baculovirus Expression Vectors - A Laboratory Manual; W.H.Freeman & Co, New York), wie des polH-Promoters oder des p10-Promoters, Zur Expression von MIA mit Hilfe des polH-Promoters wird wie folgt vorgegangen:

Ein für MIA codierendes DNA-Fragment mit Schnittstellen für die Restriktionsendonukleasen EcoRI (benachbart dem 5'-Ende des späteren MIA-Transkriptes) und PstI (benachbart dem 3'-Ende des späteren MIA-Transkriptes) wird durch PCR-Amplifikation nach bekannten Techniken unter Verwendung der MIA cDNA als Matrize und der Primer 5'-CGTGAATTCAACATGGCCCGGTCCCTGGTGTGC-3' (SEQ ID NO:21) und 5'-TATCTGCAGTCACTGGCAGTAGAAATCCCA-3' (SEQ ID NO:22) erhalten. Dieses Fragment wird mit EcoRI und PstI nachgeschnitten (zur Erzeugung der entsprechenden kohäsiven Enden) und in den mit denselben Endonukleasen restringierten Transfer-Vektor pVL1393 (O'Reilly DR, Miller LK und Luckow VA 1992; Baculovirus Expression Vectors - A Laboratory Manual; W.H.Freeman & Co, New York), der kommerziell erhältlich ist (PharMingen, San Diego, CA oder Invitrogen Corporation, San Diego, CA), einligiert. Das resultierende Transfer-Expressionsplasmid pVL1393-MIA wird zur Vermehrung in E.coli K12 transfiziert und Plasmid-DNA nach etablierten Methoden präpariert. Der Transfer der unter Kontrolle des polH-Promoters stehenden MIA-DNA vom Transfer-Plasmid in den Baculovirus-Vektor erfolgt durch homologe Rekombination nach etablierten Methoden (O'Reilly et al. 1992, siehe oben). Hierzu werden 0.5 µg BaculoGold DNA (linearisierte AcNPV Virus-DNA mit Lethal-Deletion und durch den polH-Promoter gesteuerter lacZ Expression, käuflich erhältlich bei PharMingen, Bestellnummer 21100D) und 2 µg pVL1393-MIA gemischt, 5 Minuten bei Raumtemperatur inkubiert und anschließend mit 1 ml 125 mM Hepes pH 7.1, 125 mM CaC12 , 140 mM NaCl versetzt. Diese Mischung wird zu 2 x 10⁶ SF9-Insektenzellen (Invitrogen, Bestellnummer B825-01) in einer Kulturschale von 60 mm Durchmesser gegeben, die zuvor mit 1 ml Grace's Medium mit 10 % foetalem Kälberserum überschichtet worden waren. Nach 4 Stunden Inkubation bei 4°C wird das DNA-haltige Medium entfernt und die Zellen werden in frischem Medium für 4 Tage bei 27°C inkubiert. Die auf diesem Weg erhaltenen rekombinanten Baculoviren werden anschließend zweimal über Plaque-Bildung gereinigt (O'Reilly et al. 1992, siehe oben), wobei Viren, die MIA durch homologe Rekombination insertiert haben, sich durch das Fehlen von β-Galactosidase-Aktivität (optisch erkennbar durch das Fehlen einer Blaufärbung in Gegenwart von 5-Brom-4-chlor-3-indolyl-β-D-galactopyranosid) von den verwendeten Wildtyp-Viren (AcNPV mit Lethal-Deletion und durch den polH-Promoter gesteuerter lacZ Expression, käuflich erhältlich bei PharMingen, Bestellnummer 21100D) unterscheiden. Mit einem derart erhaltenen, MIA-exprimierenden rekombinanten Baculovirus werden nach etablierten Methoden (O'Reilly et al. 1992, siehe oben) SF9-Zellen infiziert (MOI = 20 pfu/Zelle) und mindestens 36 Stunden bei 27°C in serumfreiem Medium (Cell/Perfect Bac serum free insect cell culture medium, Stratagene, Bestellnummer 205120) weiterinkubiert. Anschließend werden die Zellkulturüberstände abgenommen, im Überstand befindliche Viren durch Ultrazentrifugation entfernt (Beckmann Ti 60 Rotor, 30000 rpm) und der Überstand anschließend durch einen Microcon 100 Filter (Amicon, Ausschlußgrenze 100 kD) filtriert. Die so erhaltene MIA-haltige Lösung kann in den in Beispiel 3 und 4 beschriebenen Tests eingesetzt oder gemäß Beispiel 1 weiter aufgereinigt werden.

### Beispiel 8

### Nachweis von MIA mRNA in verschiedenen Zellen

Ein Nachweis, daß in einer bestimmten Zelle MIA exprimiert wird und demzufolge MIA mRNA vorhanden ist, kann einerseits mit den etablierten Methoden der Nucleinsäure-Hybridisierung erfolgen, wie etwa Northern-Hybridisierung, in-situ-Hybridisierung, Dot- oder Slot-Hybridisierung und daraus abgeleiteten diagnostischen Techniken (Sambrook et al. 1989, Molecular Cloning - A Laboratory Manual; Cold Spring Harbor Laboratory Press; Nucleic Acid Hybridisation - A Practical Approach, Hrsg. BD Hames und SJ Higgins (1985), IRL Press; WO 89/06698, EP-A 0 200 362, USP 2915082, EP-A 0 063 879, EP-A 0 173 251, EP-A 0 128 018). Andererseits können Methoden aus dem vielfältigen Repertoire der Amplifikationstechniken unter Verwendung von MIA-spezifischen Primern angewandt werden (PCR Protocols - A Guide to Methods and Applications; Hrsg. MA Innis, DH Gelfand, JJ Sninsky, TJ White (1990), Academic Press Inc; PCR - A Practical Approach; Hrsg. MJ McPherson, P Quirke, GR Taylor (1991), IRL Press). Die Tabellen 2A und 2B zeigen die MIA Expression in verschiedenen humanen Tumoren, Tumorlinien und normalen Zellen, die in diesem Fall durch Northern-Hybridisierung unter Verwendung der radioaktiv markiertem humanen MIA cDNA (Seq. ID No. 1) ermittelt wurde. Die RNA wurde hierfür aus den aufgelisteten Zellen nach der Methode von Chomczynski und Sacchi (Anal. Biochem. 162 (1987), 156-159) isoliert. 20 pg Gesamt-RNA wurden auf einem 1% Agarose-Formaldehyd-Gel aufgetrennt und auf Nylon-Membranen (Amersham, Braunschweig) nach Standard-Methoden übertragen (Sambrook et al. (1989); Molecular Cloning - A Laboratory Manual; Cold Spring Harbor Laboratory Press). Als Probe wurde die komplette humane MIA cDNA (Seq. ID No. 1) radioaktiv markiert (Feinberg und Vogelstein (1984), Anal. Biochem. 137, 266-267). Die Hybridisierung erfolgte bei 68°C in 5 x SSC, 5 x Denhardt, 0.5 % SDS, 10 % Dextransulfat und 100 µg/ml Lachssperma-DNA. Anschließend wurden die Membranen zweimal je eine Stunde in 1 x SSC bei 68°C gewaschen und dann auf Roentgenfilm exponiert.

**Tabelle 2A**

| Tumor | Anzahl getestet | positiv im Northern Blot |
|---|---|---|
| Astrocytom | 10 | 3 |
| Oligodendrogliom | 4 | 0 |
| Ependymom | 3 | 0 |
| Neuroblastom | 4 | 0 |
| Glioblastom | 13 | 0 |
| | | |
| Colon-CA | 2 | 1 |
| malignes Melanom ZNS-Metastase | 6 | 6 |
| | | |
| Medulloblastom | 2 | 0 |
| Mamma-CA | 1 | 0 |
| Bronchial-CA ZNS-Metastase | 2 | 0 |

**Tabelle 2B**

| Normalzellen | Anzahl getestet | positiv im Northern Blot |
|---|---|---|
| embryonale Fibroblasten | 2 | 0 |
| mononukleäre Blutzellen (3 Spender) | 3 | 0 |

### Beispiel 9

### Verwendung von MIA codierenden Nukleinsäuren zu therapeutischen Zwecken

MIA hemmt die Proliferation und die Metastasierung von Tumorzellen. Dieser Effekt kann in einem Tiermodell oder einem Patienten nicht nur durch die exogene Zufuhr von MIA-Protein hervorgerufen werden, sondern auch durch das Einbringen eines DNA-Segmentes, das entweder für MIA unter einem geeigneten Promoter codiert oder aber einen geeigneten Promoter enthält, der durch homologe Rekombination vor das zelleigene MIA Gen in das Genom integrieren kann. In letzterem Fall muß dieser Promoter von Sequenzabschnitten flankiert sein, die den Sequenzen des humanen (oder im Falle eines Tiermodells tierischen) MIA Gens im 5'-untranslatierten Bereich möglichst weitgehend homolog oder vorzugsweise identisch sind (WO 91/09955).

Mit diesem Verfahren läßt sich erreichen, daß die Tumorzelle vermehrt MIA exprimiert und so die eigene Proliferation und ihre Metastasierung inhibiert, sofern die DNA-Segmente in die Tumorzelle selbst eingebracht werden. In vielen Fällen wird es jedoch nicht erforderlich sein, die entsprechenden Gensegmente spezifisch und ausschließlich in die Tumorzellen selbst einzubringen, da auch eine Expression in anderen, vorzugsweise dem Tumor benachbarten, Körperzellen über eine vermehrte MIA-Ausschüttung eine Inhibition der Tumorzellen bewirkt. Im folgenden Beispiel ist die therapeutische Wirkung eines MIA-codierenden DNA-Segmentes in einem Tiermodell gezeigt.

Die Injektion von murinen B16-Melanom-Zellen (ATCC CRL 6322) in die Schwanzvene von C57BL-Mäusen mit nachfolgender Quantifizierung von Lungenmetastasen stellt ein etabliertes in vivo Metastasierungsmodell dar. 100.000 Zellen der Melanomlinie B16 wurden retrobulbär in C57BL-Mäuse injiziert (Tag 1, 16 Tiere) Nach 48 Stunden wurden bei 8 Tieren je 100 µg des MIA-Expressionsplasmides pCMX-PL1-MIA (Beispiel 7a) in TE (10 mM TrisCl pH 8.0, 1 mM EDTA) gemischt mit DOTAP Transfektionsreagenz (Leventis und Silvius (1990), Biochim.Biophys.Acta 1023, 124-132, kommerziell erhältlich bei Boehringer Mannheim, Cat.No 1202375), in die Schwanzvene injiziert. Die Kontrollgruppe (8 Tiere) erhielt dasselbe Plasmid, jedoch ohne MIA-Sequenzen. Nach 13 Tagen hatten in der Kontrollgruppe ohne MIA 6 von 8 Tieren einen lokalen Tumor entwickelt; die durchschnittliche Zahl der Metastasen in Lunge, Milz, Niere und Leber betrug 7.8. In der Gruppe, die das MIA codierende Plasmid erhalten hatte, entwickelten nur 4 von 8 Tieren einen lokalen Tumor und die durchschnittliche Zahl der Metastasen betrug 2.7.

## Patentansprüche

1. Protein mit einer Melanoma-inhibierenden Aktivität, welches das Wachstum der Zellinien HTZ 19-dM und ATCC CRL 1424 inhibiert und
a) codiert wird von der in SEQ ID NO:1 für das reife Protein bzw. für das Protein mit N-terminaler Prä-Sequenz gezeigten DNA-Sequenz, der in SEQ ID NO:3 gezeigten genomischen Sequenz oder
b) codiert wird von DNA-Sequenzen, die mit den in SEQ ID NO:1 oder 3 gezeigten DNA-Sequenzen oder Fragmenten dieser DNA-Sequenzen im DNA-Bereich, der für das reife Protein codiert, hybridisieren.

2. Protein nach Anspruch 1, welches eine Größe von ca. 11 kD (SDS-Page, nicht reduziert) aufweist und erhältlich ist aus dem Zellkulturüberstand der Melanomzellinie HTZ 19-dM und Aufreinigung über Gelchromatographie und reverse phase HPLC.

3. Protein nach Anspruch 1 oder 2, dadurch gekennzeichnet, daß es
a) das Produkt einer prokaryontischen oder eukaryontischen Expression einer exogenen DNA ist,
b) codiert wird von der in SEQ ID NO:1 für das reife Protein bzw. für das Protein mit N-terminaler Prä-Sequenz gezeigten DNA-Sequenz, oder der in SEQ ID NO:3 gezeigten genomischen Sequenz,
c) codiert wird von DNA-Sequenzen, die mit den in SEQ ID NO:1 oder 3 gezeigten DNA-Sequenzen oder Fragmenten dieser DNA-Sequenzen im DNA-Bereich, der für das reife Protein codiert, hybridisieren, oder
d) codiert wird von DNA-Sequenzen, die ohne die Degeneration des genetischen Codes mit den in b) bis c) definierten Sequenzen hybridisieren würden und für ein Polypeptid mit entsprechender Aminosäuresequenz codieren.

4. Protein gemäß Anspruch 1 bis 3, welches aus den Nukleotiden 40 bis 432 oder 112 bis 432 von SEQ ID NO:1 besteht.

5. Protein gemäß Anspruch 1 oder 3, welches von den Nukleotiden 110 - 499 oder 179 bis 499 von SEQ ID NO:4 codiert wird.

6. Zellinie HTZ 19-dM (DSM ACC 2133).

7. Nukleinsäure, welche für ein Protein gemäß einem der Ansprüche 1 bis 5 codiert, dadurch gekennzeichnet, daß sie ausgewählt ist aus der Gruppe
a) der in SEQ ID NO 1 oder 3 gezeigten DNA-Sequenzen oder der komplementären DNA-Sequenzen,
b) Nukleinsäure-Sequenzen, die mit einer der Sequenzen von a) hybridisieren,
c) Nukleinsäure-Sequenzen, die ohne die Degeneration des genetischen Codes mit einer der in a) oder b) genannten Sequenzen hybridisieren würden.

8. DNA mit der in SEQ ID NO:1 gezeigten Sequenz.

9. DNA mit der in SEQ ID NO:3 gezeigten Sequenz.

10. DNA mit der in SEQ ID NO:4 gezeigten Sequenz.

11. Rekombinanter Expressionsvektor, der DNA enthält, die für ein Protein nach den Ansprüchen 1 bis 5 codiert und der in einem transformierten Mikroorganismus oder einer transformierten eukaryontischen Zelle die proteincodierende DNA exprimiert.

12. Prokaryontische oder eukaryontische Wirtszelle, welche mit einer DNA, die für ein Protein nach den Ansprüchen 1 bis 5 codiert, transfiziert ist und das genannte Protein produzieren kann.

13. Wirtszelle nach Anspruch 12, die E.coli oder eine Säugerzellinie ist.

14. Verwendung einer DNA, die für ein Protein nach den Ansprüchen 1 bis 5 codiert, zur Transfektion eines prokaryontischen oder eukaryontischen Organismus.

15. Verfahren zur Gewinnung eines Proteins mit Melanom-inhibierender Aktivität durch Isolierung aus dem Kulturüberstand der Melanomzellinie HTZ 19-dM durch gelchromatographische Auftrennung und Aufreinigung einer Fraktion des Überstandes, die einem Molekulargewicht von ca. 11 kD entspricht.

16. Verfahren zur rekombinanten Herstellung eines Proteins mit Melanom-inhibierender Aktivität durch Expression einer DNA gemäß einem der Ansprüche 7 bis 10 in einer geeigneten Wirtszelle und Isolierung des Proteins aus der Wirtszelle oder dem Kulturüberstand der Wirtszelle.

17. Verwendung eines Proteins mit Melanom-inhibierender Aktivität gemäß einem der Ansprüche 1 - 5 als Antigen oder Immunogen zur Herstellung von Antikörpern, die an dieses Protein binden.

18. Antikörper gegen ein Melanom-inhibierendes Protein, erhältlich durch Immunisierung eines Tieres mit einem Protein gemäß einem der Ansprüche 1 - 5 und Gewinnung der Antikörper aus dem Serum oder Milzzellen der immunisierten Tiere.

19. Verwendung eines Melanom-inhibierenden Proteins gemäß einem der Ansprüche 1 - 5 zur Herstellung eines Therapeutikums, das Anwendung in der Tumortherapie oder als Immunsuppressivum findet.

20. Therapeutische Zusammensetzung, enthaltend ein Melanom-inhibierendes Protein gemäß einem der Ansprüche 1 - 5, ggf. zusammen mit pharmazeutischen Hilfs-, Füll- und/oder Zusatzstoffen.

21. Verfahren zum Nachweis von Nukleinsäuren, welche für ein MIA-Protein codieren, dadurch gekennzeichnet, daß die zu untersuchende Probe mit einer Nukleinsäurensonde inkubiert wird, welche ausgewählt ist aus der Gruppe
a) der in SEQ ID NO:1 und 3 gezeigten DNA-Sequenzen oder der dazu komplementären Sequenz,
b) Nukleinsäuren, die mit einer der Sequenzen von a) hybridisieren,
die Nukleinsäuresonde mit der Nukleinsäure der Probe inkubiert wird und die Hybridisierung ggf. über einen weiteren Bindepartner von Nukleinsäure der Probe und Nukleinsäuresonde nachgewiesen wird.

22. Verfahren nach Anspruch 21, dadurch gekennzeichnet, daß die nachzuweisende Nukleinsäure vor dem Nachweis amplifiziert wird.

23. Verfahren zur Herstellung eines Proteins nach den Ansprüchen 1 bis 5, durch Expression des endogenen Gens für dieses Protein in einer Säugerzelle durch Insertion eines DNA-Konstrukts in das Genom der Zelle durch homologe Rekombination, wobei dieses DNA-Konstrukt ein DNA-Regulationselement umfaßt, welches in der Lage ist, die Expression dieses Gens zu stimulieren, wenn es operativ daran gekoppelt ist, und ebenfalls umfaßt ein oder mehrere DNA-Targetsegmente, welche homolog zu einer Region in diesem Genom ist, die innerhalb oder in der Nähe des Gens liegt, Züchtung der Zellen und Gewinnung des MIA-Proteins aus den Zellen oder dem Zellkulturüberstand.

24. Verfahren zur Herstellung eines Proteins gemäß den Ansprüchen 1 bis 5, durch Expression mindestens eines exogenen Gens, welches für dieses Protein codiert, in einer Säugerzelle durch Insertion eines DNA-Konstrukts in das Genom der Zelle durch homologe Rekombination, wobei dieses DNA-Konstrukt ein DNA-Regulationselement umfaßt, welches in der Lage ist, die Expression dieses Gens zu stimulieren, wenn es operativ daran gekoppelt ist, ein DNA-Element, welches für dieses Protein codiert, und ebenfalls umfaßt ein oder mehrere DNA-Targetsegmente, welche homolog zu einer Region in diesem Genom sind, und Züchtung und Gewinnung des MIA-Proteins aus den Zellen oder dem Zellkulturüberstand.

25. Verwendung eines DNA-Konstrukts, welches durch homologe Rekombination in das Genom von Säugerzellen eingeführt werden kann, wobei dieses DNA-Konstrukt umfaßt:
- ein DNA-Regulationselement, welches in der Lage ist, die Expression eines Gens zu modulieren, wenn es daran operativ gekoppelt ist,
- wobei dieses Gen für ein Protein nach den Ansprüchen 1 bis 5 codiert,
- und ein oder mehrere DNA-Target-Segmente, welche homolog zu einer Region in diesem Genom sind, die innerhalb oder in der Nähe des genannten Gens liegen,
- und wobei dieses Konstrukt so in das Genom der Säugerzelle insertiert werden kann, daß das regulatorische Segment operativ an das Gen gekoppelt ist, welches für das Protein mit MIA-Aktivität codiert,
zur Herstellung eines Tumortherapeutikums oder Immunosuppressivums.

26. Verwendung eines DNA-Konstrukts, welches durch homologe Rekombination in das Genom von Säugerzellen eingeführt werden kann, wobei dieses DNA-Konstrukt umfaßt:
- ein DNA Regulationselement, welches in der Lage ist, die Expression eines Gens zu modulieren, wenn es daran operativ gekoppelt ist,
- wobei dieses Gen für ein Protein nach den Ansprüchen 1 bis 5 codiert,
- und ein oder mehrere DNA-Targetsegmente, welche homolog zu einer für die Insertion des Konstrukts geeigneten Region in diesem Genom sind,
- und wobei dieses Konstrukt so in das Genom der Säugerzelle insertiert werden kann, daß das regulatorische Segment die Expression des Gens in die Säugerzelle moduliert.

## Claims

1. Protein with a melanoma-inhibiting activity which inhibits the growth of the cell lines HTZ 19-dM and ATCC CRL 1424 and
a) is coded by the DNA sequence shown in SEQ ID NO 1 for the mature protein or for the protein with a N-terminal pre sequence, by the genomic sequence shown in SEQ ID NO 3, or
b) is coded by DNA sequences which hybridize with the DNA sequences shown in SEQ ID NO 1 or 3 or fragments of these DNA sequences in the DNA region which codes for the mature protein.

2. Protein as claimed in claim 1, which has a size of ca. 11 kD (SDS-Page, non-reduced) and is obtainable from the cell culture supernatant of the melanoma cell line HTZ 19-dM and purification by means of gel chromatography and reverse phase HPLC.

3. Protein as claimed in claim 1 or 2, wherein it
a) is a product of a prokaryotic or eukaryotic expression of an exogenous DNA,
b) is coded by the DNA sequence shown in SEQ ID NO 1 for the mature protein or for the protein with an N-terminal pre sequence, or by the genomic sequence shown in SEQ ID NO 3,
c) is coded by DNA sequences which hybridize with the DNA sequences shown in SEQ ID NO 1 or 3 or fragments of these DNA sequences in the DNA region which codes for the mature protein, or
d) is coded by DNA sequences which, if there was no degeneracy of the genetic code, would hybridize with the sequences defined in b) to c) and code for a polypeptide with the same amino acid sequence.

4. Protein as claimed in claims 1 to 3, which is coded by nucleotides 40 to 432 or 112 to 432 of SEQ ID NO 1.

5. Protein as claimed in claim 1 or 3, which is coded by nucleotides 110 - 499 or 179 - 499 of SEQ ID NO 4.

6. Cell line HTZ 19-dM (DSM ACC 2133).

7. Nucleic acid which codes for a protein as claimed in one of the claims 1 to 5, wherein it is selected from the group
a) DNA sequences shown in SEQ ID NO 1 or 3 or the complementary DNA sequences,
b) nucleic acid sequences which hybridize with one of the sequences from a),
c) nucleic acid sequences which, if there was no degeneracy of the genetic code, would hybridize with one of the sequences stated in a) or b).

8. DNA having the sequence shown in SEQ ID NO 1.

9. DNA having the sequence shown in SEQ ID NO 3.

10. DNA having the sequence shown in SEQ ID NO 4.

11. Recombinant expression vector containing DNA which codes for a protein as claimed in claims 1 to 5 and which expresses the protein coding DNA in a transformed microorganism or a transformed eukaryotic cell.

12. A prokaryotic or eukaryotic host cell which is transfected with a DNA which codes for a protein as claimed in claims 1 - 5 and which can produce the said prptein.

13. The host cell as claimed in claim 12, which is E. coli or a mammalian cell line.

14. The use of a DNA which codes for a protein as claimed in claims 1 to 5, for transfection of a prokaryotic or eukaryotic organism.

15. Process for obtaining a protein with melanoma-inhibiting activity by isolation from the culture supernatant of the melanoma cell line HTZ 19-dM by gel chromatographic separation and purifying a fraction of the supernatant that corresponds to a molecular weight of ca. 11 kD.

16. Process for the recombinant production of a protein with melanoma-inhibiting activity by expression of a DNA as claimed in one of the claims 7 to 10 in a suitable host cell and isolation of the protein from the host cell or the culture supernatant of the host cell.

17. Use of a protein with melanoma-inhibiting activity as claimed in one of the claims 1 - 5 as an antigen or immunogen for the production of antibodies which bind to this protein.

18. Antibody against a melanoma-inhibiting protein obtainable by immunizing an animal with a protein as claimed in one of the claims 1 - 5 and isolating the antibodies from the serum or spleen cells of the immunized animals.

19. Use of a melanoma-inhibiting protein as claimed in claims 1 - 5 for the production of a therapeutic agent which can be used in tumour therapy or as an immunosuppressive agent.

20. Therapeutic composition containing a melanoma-inhibiting protein as claimed in one of the claims 1 - 5, if desired, together with pharmaceutical auxiliary substances, fillers and/or additives.

21. Method for the detection of nucleic acids that code for a MIA protein, wherein the sample to be examined is incubated with a nucleic acid probe which is selected from the group
a) DNA sequences shown in SEQ ID NO 1 and 3 or their complementary sequences,
b) nucleic acids which hybridize with one of the sequences from a),
the nucleic acid probe is incubated with the nucleic acid of the sample and the hybridization of the nucleic acid of the sample and the nucleic acid probe is detected, if desired via a further binding partner.

22. Method as claimed in claim 21, wherein the nucleic acid to be detected is amplified before detection.

23. Process for the production of a protein according to claims 1 to 5, comprising the steps of expressing in a mammalian cell the endogenous gene for this protein by inserting a DNA construct into the genome of the cell by homologous recombination, said DNA construct comprising a DNA regulatory element capable of stimulating the expression of said gene if operatively linked thereto, and further comprising one or more DNA target segments homologous to a region in this genome, which region is within or proximal to said gene; culturing the cells and recovering the MIA protein from the cells or the cell culture supernatant.

24. A process for the production of a protein as claimed in claims 1 to 5, comprising the steps of expressing in a mammalian cell at least one exogenous gene which codes for this protein by inserting a DNA construct into the genome of the cell by homologous recombination, said DNA construct comprising a DNA regulatory element capable of stimulating the expression of said gene if operatively linked thereto, a DNA element which codes for said protein and further comprising one or more DNA target segments homologous to a region in this genome, culturing the cells and recovering the MIA protein from the cells or cell culture supernatant.

25. The use of a DNA construct which can be introduced into the genome of mammalian cells by homologous recombination, said DNA construct comprising
a DNA regulatory element capable of modulating the expression of a gene if operatively linked thereto,
said gene coding for a protein according to claims 1 to 5;
and one or more DNA target segments homologous to a region in this genome, which region is within or proximal to said gene;
and wherein said construct can be inserted into said genome of the mammalian cell in such fashion that the regulatory segment is operatively linked to the gene coding for the protein having MIA activity,
for preparing a tumour therapeutic agent or immunosuppressive agent.

26. The use of a DNA construct which can be introduced into the genome of mammalian cells by homologous recombination, said DNA construct comprising
a DNA regulatory element capable of modulating the expression of a gene if operatively linked thereto,
said gene coding for a protein according to claims 1 to 5,
and one or more DNA target segments homologous to a region in this genome where the insertion of the construct is appropriate,
and wherein said construct can be inserted into said genome of the mammalian cell in such a fashion that the regulatory segment modulates the expression of said gene in said mammalian cell.

## Revendications

1. Protéine ayant une activité inhibitrice de mélanome, qui inhibe la croissance des lignées cellulaires HTZ 19-dM et ATCC CRL 1424, et qui est
a) codée par la séquence d'ADN indiquée dans SEQ ID N°:1 pour la protéine mature ou pour la protéine ayant une pré-séquence N-terminale, la séquence génomique indiquée dans SEQ ID N°:3, ou
b) codée par les séquences d'ADN qui s'hybrident aux séquences d'ADN indiquées dans SEQ ID N°:1 ou 3 ou aux fragments de ces séquences d'ADN dans la région d'ADN qui code pour la protéine mature.

2. Protéine selon la revendication 1, qui présente une taille d'environ 11 kD (SDS-Page, non réduit) et qui peut être obtenue à partir du surnageant de culture cellulaire de la lignée cellulaire de mélanome HTZ 19-dM, après purification par chromatographie sur gel et HPLC à phases inversées.

3. Protéine selon la revendication 1 ou 2, caractérisée par le fait qu'elle est
a) le produit d'une expression procaryote ou eucaryote d'un ADN exogène,
b) codée par la séquence d'ADN indiquée dans SEQ ID N°:1 pour la protéine mature ou pour la protéine ayant une pré-séquence N-terminale, ou par la séquence génomique indiquée dans SEQ ID N°:3,
c) codée par les séquences d'ADN qui s'hybrident aux séquences d'ADN indiquées dans SEQ ID N°:1 ou 3 ou aux fragments de ces séquences d'ADN dans la région d'ADN qui code pour la protéine mature, ou
d) codée par les séquences d'ADN qui, sans la dégénérescence du code génétique, se seraient hybridées aux séquences définies dans b) à c) et qui codent pour un polypeptide ayant la séquence d'acides aminés correspondante.

4. Protéine selon les revendications 1 à 3, qui est constituée des nucléotides 40 à 432 ou 112 à 432 de la SEQ ID N°:1.

5. Protéine selon la revendication 1 ou 3, qui est codée par les nucléotides 110 à 499 ou 179 à 499 de la SEQ ID N°:4.

6. Lignée cellulaire HTZ 19-dM (DSM ACC 2133).

7. Acide nucléique qui code pour une protéine selon l'une quelconque des revendications 1 à 5, caractérisé par le fait qu'il est choisi dans le groupe constitué par
a) les séquences d'ADN indiquées dans SEQ ID N°:1 ou 3 ou les séquences d'ADN complémentaires,
b) les séquences d'acides nucléiques qui s'hybrident à une des séquences de a),
c) des séquences d'acides nucléiques qui, sans la dégénérescence du code génétique, se seraient hybridées à une des séquences mentionnées dans a) ou b).

8. ADN ayant la séquence définie dans SEQ ID N°:1.

9. ADN ayant la séquence définie dans SEQ ID N°:3.

10. ADN ayant la séquence définie dans SEQ ID N°:4.

11. Vecteur d'expression recombinant contenant de l'ADN qui code pour une protéine selon les revendications 1 à 5 et qui, dans un micro-organisme transformé ou dans une cellule eucaryote transformée, exprime l'ADN codant pour la protéine.

12. Cellule hôte procaryote ou eucaryote, qui est transfectée avec un ADN qui code pour une protéine selon les revendications 1 à 5 et qui peut produire la protéine mentionnée.

13. Cellule hôte selon la revendication 12, qui est E. coli ou une lignée cellulaire de mammifère.

14. Utilisation d'un ADN qui code pour une protéine selon les revendications 1 à 5, pour la transfection d'un organisme procaryote ou eucaryote.

15. Procédé d'obtention d'une protéine ayant une activité inhibitrice de mélanome, par isolement à partir du surnageant de culture de la lignée cellulaire de mélanome HTZ 19-dM, par séparation par chromatographie sur gel et purification d'une fraction du surnageant, qui présente un poids moléculaire d'environ 11 kD.

16. Procédé de préparation recombinante d'une protéine ayant une activité inhibitrice de mélanome, par expression d'un ADN selon l'une quelconque des revendications 7 à 10 dans une cellule hôte appropriée, et isolement de la protéine à partir de la cellule hôte ou du surnageant de culture de la cellule hôte.

17. Utilisation recombinante d'une protéine ayant une activité inhibitrice de mélanome selon l'une quelconque des revendications 1-5, comme antigène ou immunogène pour la préparation d'anticorps qui se lient à cette protéine.

18. Anticorps dirigé contre une protéine inhibitrice de mélanome, que l'on peut obtenir par immunisation d'un animal avec une protéine selon l'une quelconque des revendications 1-5 et obtention de l'anticorps à partir du sérum ou des cellules de la rate de l'animal immunisé.

19. Utilisation d'une protéine inhibitrice de mélanome selon l'une quelconque des revendications 1-5 pour la préparation d'un agent thérapeutique qui est utilisable dans le traitement thérapeutique de tumeurs ou comme immunosuppresseur.

20. Composition thérapeutique contenant une protéine inhibitrice de mélanome selon l'une quelconque des revendications 1-5, éventuellement avec des adjuvants, des charges et/ou des additifs pharmaceutiques.

21. Procédé de détection d'acides nucléiques qui codent pour une protéine MIA (inhibitrice de mélanome), caractérisé par le fait que l'échantillon à analyser est incubé avec une sonde d'acide nucléique choisie dans le groupe constitué par
a) les séquences d'ADN indiquées dans SEQ ID N°:1 et 3 ou les séquences complémentaires,
b) les acides nucléiques qui s'hybrident à une des séquences de a),
la sonde d'acide nucléique est incubée avec l'acide nucléique de l'échantillon et l'hybridation est éventuellement détectée par l'intermédiaire d'un partenaire de liaison des acides nucléiques de l'échantillon et de la sonde d'acide nucléique.

22. Procédé selon la revendication 21, caractérisé par le fait que l'acide nucléique à détecter est amplifié avant la détection.

23. Procédé de préparation d'une protéine selon l'une quelconque des revendications 1-5, par expression du gène endogène codant pour cette protéine dans une cellule de mammifère, par insertion d'une construction d'ADN dans le génome de la cellule par recombinaison homologue, cette construction d'ADN englobant un élément de régulation d'ADN qui est capable de stimuler l'expression de ce gène lorsqu'il est couplé à celui-ci de manière opérationnelle, et englobant également un ou plusieurs segments cibles d'ADN qui sont homologues d'une région de ce génome, situé à l'intérieur ou à proximité du gène, culture des cellules et récupération de la protéine MIA à partir des cellules ou du surnageant de culture.

24. Procédé de préparation d'une protéine selon l'une quelconque des revendications 1 à 5, par expression d'au moins un gène exogène qui code pour cette protéine, dans une cellule de mammifère par insertion d'une construction d'ADN dans le génome de la cellule par recombinaison homologue, cette construction d'ADN englobant un élément de régulation d'ADN qui est capable de stimuler l'expression de ce gène lorsqu'il est couplé à celui-ci de manière opérationnelle, un élément d'ADN qui code pour cette protéine, et englobant également un ou plusieurs segments cibles d'ADN qui sont homologues d'une région de ce génome, et culture et récupération de la protéine MIA à partir des cellules ou du surnageant de culture.

25. Utilisation d'une construction d'ADN qui peut être introduite par recombinaison homologue dans le génome de cellules de mammifère, cette construction d'ADN englobant
- un élément de régulation d'ADN qui est capable de moduler l'expression d'un gène lorsqu'il est couplé à celui-ci de manière opérationnelle,
- ce gène codant pour une protéine selon l'une quelconque des revendications 1 à 5,
- et un ou plusieurs segments cibles d'ADN qui sont homologues d'une région de ce génome, qui sont situés à l'intérieur ou à proximité du gène mentionné,
- et cette construction pouvant être insérée dans le génome de la cellule de mammifère de telle façon que le segment régulateur soit couplé de manière opérationnelle au gène codant pour la protéine ayant une activité MIA,
pour la préparation d'un agent de traitement thérapeutique des tumeurs, ou d'immunosuppresseurs.

26. Utilisation d'une construction d'ADN qui peut être introduite par recombinaison homologue dans le génome de cellules de mammifère, cette construction d'ADN englobant
- un élément de régulation d'ADN qui est capable de moduler l'expression d'un gène lorsqu'il est couplé à celui-ci de manière opérationnelle,
- ce gène codant pour une protéine selon l'une quelconque des revendications 1 à 5,
- et un ou plusieurs segments d'ADN cible qui sont homologues d'une région appropriée pour l'insertion de la construction dans ce génome,
- et cette construction pouvant être insérée dans le génome de la cellule de mammifère de telle façon que le segment régulateur module l'expression du gène dans la cellule de mammifère.


### SEQUENZPROTOKOLL

### (1) ALGEMEINE INFORMATION:

(i) ANMELDER:
   (A) NAME: BOEHRINGER MANNHEIM GMBH
   (B) STRASSE: Sandhofer Str. 116
   (C) ORT: Mannheim
   (D) BUNDESLAND: BW
   (E) LAND: Germany
   (F) POSTLEITZAHL: D-68305
   (G) TELEPHON: 08856/60-3446
   (H) TELEFAX: 08856/60-3451
(ii) ANMELDETITEL: Melanom-inhibierendes Protein
(iii) ANZAHL DER SEQUENZEN: 24
(iv) COMPUTER-LESBARE FORM:
   (A) DATENTRÄGER: Floppy disk
   (B) COMPUTER: IBM PC compatible
   (C) BETRIEBSSYSTEM: PC-DOS/MS-DOS
   (D) SOFTWARE: PatentIn Release #1.0, Version #1.25 (EPA)
(vi) FRÜHERE ANMELDEDATEN:
   (A) ANMELDENUMMER: DE P 43 24 247.2
   (B) ANMELDEDATUM: 20-JUL-1993

### (2) INFORMATION ZU SEQ ID NO: 1:

(i) SEQUENZ CHARAKTERISTIKA:
   (A) LÄNGE: 459 Basenpaare
   (B) ART: Nukleinsäure
   (C) STRANGFORM: Einzel
   (D) TOPOLOGIE: linear
(ii) ART DES MOLEKÜLS: cDNS
(ix) MERKMALE:
   (A) NAME/SCHLÜSSEL: CDS
   (B) LAGE: 40..432
(ix) MERKMALE:
   (A) NAME/SCHLÜSSEL: sig_peptide
   (B) LAGE: 40..111
(ix) MERKMALE:
   (A) NAME/SCHLÜSSEL: mat_peptide
   (B) LAGE: 112..432
(xi) SEQUENZBESCHREIBUNG: SEQ ID NO: 1:
   CCAGCACCCC CTTGCTCACT CTCTTGCTCA CAGTCCACG ATG GCC CGG TCC CTG      54
                                             Met Ala Arg Ser Leu
                                             -24             -20
   GTG TGC CTT GGT GTC ATC ATC TTG CTG TCT GCC TTC TCC GGA CCT GGT    102
   Val Cys Leu Gly Val Ile Ile Leu Leu Ser Ala Phe Ser Gly Pro Gly
                   -15                 -10                 -5
   GTC AGG GGT GGT CCT ATG CCC AAG CTG GCT GAC CGG AAG CTG TGT GCG    150
   Val Arg Gly Gly Pro Met Pro Lys Leu Ala Asp Arg Lys Leu Cys Ala
                   1               5                   10
   GAC CAG GAG TGC AGC CAC CCT ATC TCC ATG GCT GTG GCC CTT CAG GAC    198
   Asp Gln Glu Cys Ser His Pro Ile Ser Met Ala Val Ala Leu Gln Asp
       15                  20                  25
   TAC ATG GCC CCC GAC TGC CGA TTC CTG ACC ATT CAC CGG GGC CAA GTG    246
   Tyr Met Ala Pro Asp Cys Arg Phe Leu Thr Ile His Arg Gly Gln Val
   30                  35                  40                  45
   GTG TAT GTC TTC TCC AAG CTG AAG GGC CGT GGG CGG CTC TTC TGG GGA    294
   Val Tyr Val Phe Ser Lys Leu Lys Gly Arg Gly Arg Leu Phe Trp Gly
                   50                  55                  60
   GGC AGC GTT CAG GGA GAT TAC TAT GGA GAT CTG GCT GCT CGC CTG GGC    342
   Gly Ser Val Gln Gly Asp Tyr Tyr Gly Asp Leu Ala Ala Arg Leu Gly
               65                  70                  75
   TAT TTC CCC AGT AGC ATT GTC CGA GAG GAC CAG ACC CTG AAA CCT GGC    390
   Tyr Phe Pro Ser Ser Ile Val Arg Glu Asp Gln Thr Leu Lys Pro Gly
           80                  85                  90
   AAA GTC GAT GTG AAG ACA GAC AAA TGG GAT TTC TAC TGC CAG            432
   Lys Val Asp Val Lys Thr Asp Lys Trp Asp Phe Tyr Cys Gln
       95                  100                 105
   TGAGCTCAGC CTACCGCTGG CCCTGCC                                      459

### (2) INFORMATION ZU SEQ ID NO: 2:

(i) SEQUENZ CHARAKTERISTIKA:
   (A) LÄNGE: 131 Aminosäuren
   (B) ART: Aminosäure
   (D) TOPOLOGIE: linear
(ii) ART DES MOLEKÜLS: Protein
(xi) SEQUENZBESCHREIBUNG: SEQ ID NO: 2:
   Met Ala Arg Ser Leu Val Cys Leu Gly Val Ile Ile Leu Leu Ser Ala
   -24             -20                 -15                 -10
   Phe Ser Gly Pro Gly Val Arg Gly Gly Pro Met Pro Lys Leu Ala Asp
               -5                  1               5
   Arg Lys Leu Cys Ala Asp Gln Glu Cys Ser His Pro Ile Ser Met Ala
       10                  15                  20
   Val Ala Leu Gln Asp Tyr Met Ala Pro Asp Cys Arg Phe Leu Thr Ile
   25                  30                  35                  40
   His Arg Gly Gln Val Val Tyr Val Phe Ser Lys Leu Lys Gly Arg Gly
                   45                  50                  55
   Arg Leu Phe Trp Gly Gly Ser Val Gln Gly Asp Tyr Tyr Gly Asp Leu
               60                  65                  70
   Ala Ala Arg Leu Gly Tyr Phe Pro Ser Ser Ile Val Arg Glu Asp Gln
           75                  80                  85
   Thr Leu Lys Pro Gly Lys Val Asp Val Lys Thr Asp Lys Trp Asp Phe
       90                  95                  100
   Tyr Cys Gln
   105

### (2) INFORMATION ZU SEQ ID NO: 3:

(i) SEQUENZ CHARAKTERISTIKA:
   (A) LÄNGE: 3565 Basenpaare
   (B) ART: Nukleinsäure
   (C) STRANGFORM: Einzel
   (D) TOPOLOGIE: linear
(ii) ART DES MOLEKÜLS: DNS (genomisch)
(ix) MERKMALE:
   (A) NAME/SCHLÜSSEL: sig_peptide
   (B) LAGE: 1378..1449
(ix) MERKMALE:
   (A) NAME/SCHLÜSSEL: exon
   (B) LAGE: 1378..1504
(ix) MERKMALE:
   (A) NAME/SCHLÜSSEL: exon
   (B) LAGE: 1586..1719
(ix) MERKMALE:
   (A) NAME/SCHLÜSSEL: exon
   (B) LAGE: 2804..2914
(ix) MERKMALE:
   (A) NAME/SCHLÜSSEL: exon
   (B) LAGE: 3232..3252
(ix) MERKMALE:
   (A) NAME/SCHLÜSSEL: -
   (B) LAGE: one-of(2216)
   (D) SONSTIGE ANGABEN: /note= "N in Position 2216 steht fuer eine unbestimmte Anzahl und Sequenz von Nukleotiden"
(xi) SEQUENZBESCHREIBUNG: SEQ ID NO: 3:
   TCTAGACANA TAAAAATAAA AGAAATCATC CAAGAATGGT GACTTGCCTA CTATTCTACT    60
   CGAGAGGCTG AGAGGGGAGG ATTTCTTGAC CCCGGNAGTT TAAGGATGCA GTGAGCTATG   120
   ATCACATCAC TGTACTTCAG CCTGAGCAAC AGCAAGATCC TGTCTCTAAA AATTAAATAA   180
   GGCTGGGCTT GGTGGCTCAT GCTGTAATCC CAGCACTTTG GAAGGCCATG GTGGGCAGAT   240
   TGCTTGAGCC CAGGAGTTTG AGACGAGGCT GGGCAACATG ACGAAACCCC GGCTCTACCA   300
   AAAAATACAA AAAATTAACT GGGCATAATG GTACATGTCT GTGGTCCCAG CTACTCGGTA   360
   GGCTGAGGTG GGAGGAATGC TTGAGCCCAG GAAATAGGGG CTACAGTGAA CCAGGATGAT   420
   GCCAGTGCAC TCCAACCTGG GCAACAGAGC AAGACTCTAC CTCAAAATAA TTTAAAAAAA 480
   TGGATTAATT GGGCATAGGT GGCTTGTGCC TGTAGTCCCA GTTACTCAGG AGCCTGAGGT 540
   GGGAGGATTG CCTGAGTCTA GGAGGTTGAG GCTGCAGTGA GCCGGGATGG CACCATTGCA 600
   CTCCACCTGG GCAACAGGGT GAGACCCTGT CTCAAAAAAG AAAAAAAAGG GAGGGGTTAT 660
   AATCACTCCT CCTGACATGA TACAGAGTAT CCATTTGAGT TCATAACATA AATATGTACT 720
   TGGTGAATGC TCTGTAACTA TTGGTGAATG CTCTGTAACT ATTGGCTTTT TTATTGTTCC 780
   CATTTTACAT ATAAGGAAGC TGAGGCTTTG TGAGGAGAAA TAGCTTAGCC CAGGTCATCC 840
   AGTGGGAAGC GTCTGGTGCA GAGGAATAGT GATCAGGGTG GGACTTTGCC TAGCCTAAGG 900
   TTCAGCATAC AATATTCAGT CAGTACTCAA GGGCTGGGCT GTTTCTGGTA ATCAAAGGGC 960
   CTGCCTTGTC CTCCTCCCCC ACAGCAGGAA ATTCCAAGGT GGTTTTCTTT ACAGGCTCCT 1020
   CCGCTTCTGT GGCCAGAGGG GACAGCGGAG GACCCCAGGT ACCTAAGCCA ACTCAAGAGA 1080
   AGATGGAATT GAATATTTCA ACCACCTTAT CTAGGCCTCT GTGATTGTTG AGGAGGGGGC 1140
   TGTCACTGGG AAAGTTGTGA GCTGCTTTGG ACCTTATCTG GGAATTTCCT TGGGCCTTAC 1200
   AGCTTTACCC TATCCTTGAA ATGGTTCTGG TTTCATAGCA ACTTCTAGGT GGTGTGGGCG 1260
   AAGTTTGGGA CTGGTTTAGG GCGGGGACAA GACCAAGAAC ACAAGTTTCC TTGTACGGGA 1320
   GAGAGGAAAT TGGAGACCCC AGCACCCCCT TGCTCACTCT CTTGCTCACA GTCCACGATG 1380
   GCCCGGTCCC TGGTGTGCCT TGGTGTCATC ATCTTGCTGT CTGCCTTCTC CGGACCTGGT 1440
   GTCAGGGGTG GTCCTATGCC CAAGCTGGCT GACCGGAAGC TGTGTGCGGA CCAGGAGTGC 1500
   AGCCGTAAGA ATGGGGAGGG GTAGAATTGG GCTTGGGTGT TAGCCTGTGT GGATGTGCTG 1560
   CATTCCCCTT CTATTCCTTC CCTAGACCCT ATCTCCATGG CTGTGGCCCT TCAGGACTAC 1620
   ATGGCCCCCG ACTGCCGATT CCTGACCATT CACCGGGGCC AAGTGGTGTA TGTCTTCTCC 1680
   AAGCTGAAGG GCCGTGGGCG GCTCTTCTGG GGAGGCAGCG TGCGTCTTGG GAGAGTGAAA 1740
   GAGGGAAGGG TACAGAGCTG GGGTAGACTC ATTATCCCCA TGAAGGGAAG ATTTGAGGGG 1800
   GGTGAACTGA AATAGACATT GTGGGGGGAT ATTGTTACTT ACTTTATTTT ATTTGCTTAT 1860
   TATTTTTTAA TTTTTTCCGA GACAGAGTCT TGCTCTGTCA CCCAGGCTGG ATGCAATGGC 1920
   ACGATCTCGG CTCACTGTAA CCTCCACCTC TTGGGTTTAA GCGATTCTCC AGCCTCAGCC 1980
   TCCCAAGTAC CTGGGATTAC AGGCATGCAC CACCACACCT NNTAATTTTT GTATTTTTAG 2040
   TAGAGACAGG GTTTTACCAT ATTGGCCAGG CTGGTCTTGA ACTCCTGACC TCATGATCTG 2100
   CCCGCCTTGG CTCCCGGAGT GCTGGGATTA CAGGTGTGAG CCACTGGCCC CCCAGCCTAT 2160
   TTTCACTTTA TTTACCAATT TTAGGACCTG ATATGGTCCC ANNNTCTGTT CTAGANTCTA 2220
   GACACCAAGA TACAACAACA AATGATCCTT TTTATTCTAA TGGAGGGAAA TGAACAAAAA 2280
   GCAAGGCATA AAAAATAGCA GCAGCCGGGC ACAGTAGCTC ACACCTGTAA TCCCAAGTAA 2340
   GGCCAAGTNN GGAGGATAGC TTGAGCCCAG GAGTTCGAGA CCAGCCTGGG CAACATAGCA 2400
   AGACCCCCAT CTCTATAAAA AAAAATTTAA AATTAACTGG GCATCATGGC ATGTGTCTGT 2460
   GGTCCCGGCT ACTCGGGAGG CTGAGGTGGG AGGATTGCTT GATCCCAGAA GTTGAGGCTG 2520
   CAGTGAGCCG TGATCATGCT ACTGCACCTC AACCTGGCCG ACACAATGAG ACCCTGTTTC 2580
   CAAAATAATA ATAATAAAAG CAAATATGCG CTGCTGTGAG AATTAACAGA GACTTACTTG 2640
   GGTGTTCAGA AAGGGCCTCT GAACAGGTGG CATTTAAGCT GAGATTCATA TGACAAGGAT 2700
   GGAGCAGTTA TGTGGAGATC AGGGAGAGGG GAGAATGCAA AGGCCTTCAG CAGGCACAAG 2760
   CTTGCCATCT TCCAGACCCT AGCTTTTAAC TCCTCTTCCC CAGGTTCAGG GAGATTACTA 2820
   TGGAGATCTG GCTGCTCGCC TGGGCTATTT CCCCAGTAGC ATTGTCCGAG AGGACCAGAC 2880
   CCTGAAACCT GGCAAAGTCG ATGTGAAGAC AGACGTGAGT GTCATGGGGG CTGGCAAGAA 2940
   ATGTGGGGGG AGGACCCTTA GGTTGTGGGG ATGGGCAAAA ATGCTCCCAC ACTTGGCTCC 3000
   CTGGCCGCCT AGGTATGTGC GCTGGGAGAA ATTCTTTCCC TGCCTCAATT TTCTCACCAG 3060
   TAAAATGGGT CCAGTTGGGA GGTGCAAAGA TTAGAGGGCT CTAGGCTAAT TTGCATAGCA 3120
   NNTGTGTGGC CAGACCTGGG CCCTGCAGCT GCAGCCTTTG CTAAAACCAC TAGATCCTTT 3180
   GTGGTGTGAC CGCTGGTTTT CTTTCCACTG TTTCCCCTTT CTCTTTTTCA GAAATGGGAT 3240
   TTCTACTGCC AGTGAGCTCA GCCTACCGCT GGCCCTGCCG TTTCCCCTCC TTGGGTTTAT 3300
   GCAAATACAA TCAGCCCAGT GCAAACGGCT CGTCTCCGTG GTCTTTGGGG TGGGGTAGGG 3360
   TAGGGTGGGG ACTGTACAAA TGAAATGTTT CTCTAGGTTG CTGAATCTAA CCAATTAACC 3420
   CGCTGCCTGT GGTAACGTCA GTGGTTGCTA GGCAGAGTTT CGCTGATGAA AGCCCTGTGC 3480
   AGTAGGAGCG CTCCTAAGCT TAGGTTTCGA CACAAGCAAA GAAAACCTAA GCAGCCCAAC 3540
   TAGGGATTGT AGTGTCCTCT CTAGA                                       3565

### (2) INFORMATION ZU SEQ ID NO: 4:

(i) SEQUENZ CHARAKTERISTIKA:
   (A) LÄNGE: 581 Basenpaare
   (B) ART: Nukleinsäure
   (C) STRANGFORM: Einzel
   (D) TOPOLOGIE: linear
(ii) ART DES MOLEKÜLS: cDNS
(ix) MERKMALE:
   (A) NAME/SCHLÜSSEL: CDS
   (B) LAGE: 110..499
(ix) MERKMALE:
   (A) NAME/SCHLÜSSEL: sig_peptide
   (B) LAGE: 110..178
(ix) MERKMALE:
   (A) NAME/SCHLÜSSEL: mat_peptide
   (B) LAGE: 179..499
(xi) SEQUENZBESCHREIBUNG: SEQ ID NO: 4:
   AAGCGGCGGA GACAGGATCG AGAACACAGG TTTCCTTGAT ATTCAGCCTG GAAGGAGGGC    60
   AGGAGGAGCC CAGAGACCTC GTTCTTCACT TGGTCATTCT CAGTCCATG ATG GTG       115
                                                         Met Val
                                                         -23
   TGG TCC CCA GTG CTC CTT GGC ATC GTC GTC TTG TCT GTT TTT TCA GGG     163
   Trp Ser Pro Val Leu Leu Gly Ile Val Val Leu Ser Val Phe Ser Gly
       -20                 -15                 -10
   CCT AGC AGG GCT GAT CGA GCT ATG CCC AAG CTG GCT GAC TGG AAG CTG     211
   Pro Ser Arg Ala Asp Arg Ala Met Pro Lys Leu Ala Asp Trp Lys Leu
   -5                   1               5                   10
   TGT GCG GAC GAG GAA TGC AGC CAT CCT ATC TCC ATG GCT GTG GCC CTC     259
   Cys Ala Asp Glu Glu Cys Ser His Pro Ile Ser Met Ala Val Ala Leu
               15                  20                  25
   CAG GAC TAC GTG GCC CCT GAT TGC CGC TTC TTG ACT ATA TAT AGG GGC     307
   Gln Asp Tyr Val Ala Pro Asp Cys Arg Phe Leu Thr Ile Tyr Arg Gly
           30                  35                  40
   CAA GTG GTG TAT GTC TTC TCC AAG TTG AAG GGC CGT GGG CGC CTT TTC     355
   Gln Val Val Tyr Val Phe Ser Lys Leu Lys Gly Arg Gly Arg Leu Phe
       45                  50                  55
   TGG GGA GGC AGT GTT CAG GGA GGT TAC TAT GGA GAC CTG GCA GCC CGC     403
   Trp Gly Gly Ser Val Gln Gly Gly Tyr Tyr Gly Asp Leu Ala Ala Arg
   60                  65                  70                  75
   CTG GGC TAT TTC CCC AGT AGC ATT GTC CGG GAG GAC CTG AAC TCG AAA     451
   Leu Gly Tyr Phe Pro Ser Ser Ile Val Arg Glu Asp Leu Asn Ser Lys
                   80                  85                  90
   CCT GGC AAA ATT GAT ATG AAG ACC GAT CAA TGG GAT TTC TAC TGC CAG     499
   Pro Gly Lys Ile Asp Met Lys Thr Asp Gln Trp Asp Phe Tyr Cys Gln
               95                  100                 105
   TGAGCTCAGC CTACCGCTAT CCCTGCAGTT ACCTTCCGGC TCTATGCAAA TACAGCAGCC   559
   AATGGCAAAA AAAAAAAAAA AA                                            581

### (2) INFORMATION ZU SEQ ID NO: 5:

(i) SEQUENZ CHARAKTERISTIKA:
   (A) LÄNGE: 130 Aminosäuren
   (B) ART: Aminosäure
   (D) TOPOLOGIE: linear
(ii) ART DES MOLEKÜLS: Protein
(xi) SEQUENZBESCHREIBUNG: SEQ ID NO: 5:
   Met Val Trp Ser Pro Val Leu Leu Gly Ile Val Val Leu Ser Val Phe
   -23         -20                 -15                 -10
   Ser Gly Pro Ser Arg Ala Asp Arg Ala Met Pro Lys Leu Ala Asp Trp
           -5                  1               5
   Lys Leu Cys Ala Asp Glu Glu Cys Ser His Pro Ile Ser Met Ala Val
    10                 15                  20                  25
   Ala Leu Gln Asp Tyr Val Ala Pro Asp Cys Arg Phe Leu Thr Ile Tyr
                   30                  35                  40
   Arg Gly Gln Val Val Tyr Val Phe Ser Lys Leu Lys Gly Arg Gly Arg
               45                  50                  55
   Leu Phe Trp Gly Gly Ser Val Gln Gly Gly Tyr Tyr Gly Asp Leu Ala
           60                  65                  70
   Ala Arg Leu Gly Tyr Phe Pro Ser Ser Ile Val Arg Glu Asp Leu Asn
       75                  80                  85
   Ser Lys Pro Gly Lys Ile Asp Met Lys Thr Asp Gln Trp Asp Phe Tyr
    90                 95                  100                 105
   Cys Gln

### (2) INFORMATION ZU SEQ ID NO: 6:

(i) SEQUENZ CHARAKTERISTIKA:
   (A) LÄNGE: 31 Basenpaare
   (B) ART: Nukleinsäure
   (C) STRANGFORM: Einzel
   (D) TOPOLOGIE: linear
(ii) ART DES MOLEKÜLS: cDNS
(ix) MERKMALE:
   (A) NAME/SCHLÜSSEL: -
   (B) LAGE: one-of(14, 17, 20)
   (D) SONSTIGE ANGABEN: /label= N /note= "N steht fuer I (Inosin)"
(xi) SEQUENZBESCHREIBUNG: SEQ ID NO: 6:
   TGTGAATTCA GTTNWSNGCN GAYCARGART G                    31

### (2) INFORMATION ZU SEQ ID NO: 7:

(i) SEQUENZ CHARAKTERISTIKA:
   (A) LÄNGE: 33 Basenpaare
   (B) ART: Nukleinsäure
   (C) STRANGFORM: Einzel
   (D) TOPOLOGIE: linear
(ii) ART DES MOLEKÜLS: cDNS
(xi) SEQUENZBESCHREIBUNG: SEQ ID NO: 7:
   TGTGTCGACT GTTCGTAGAA RTCCCATCTT RTC                33

### (2) INFORMATION ZU SEQ ID NO: 8:

(i) SEQUENZ CHARAKTERISTIKA:
   (A) LÄNGE: 305 Basenpaare
   (B) ART: Nukleinsäure
   (C) STRANGFORM: Einzel
   (D) TOPOLOGIE: linear
(ii) ART DES MOLEKÜLS: cDNS
(ix) MERKMALE:
   (A) NAME/SCHLÜSSEL: misc_RNA
   (B) LAGE: join(1..29, 277..305)
   (D) SONSTIGE ANGABEN: /function= "Primer"
(xi) SEQUENZBESCHREIBUNG: SEQ ID NO: 8:
   GAATTCAAGT TTTCGGCGGA TCAGGAGTGC AGCCACCCTA TCTCCATGGC TGTGGCCCTT  60
   CAGGACTACA TGGCCCCCGA CTGCCGATTC CTGACCATTC ACCGGGGCCA AGTGGTGTAT 120
   GTCTTCTCCA AGCTGAAGGG CCGTGGGCGG CTCTTCTGGG GAGGCAGCGT TCAGGGAGAT 180
   TACTATGGAG ATCTGGTCGC TCGCCTGGGC TATTTCCCCA GTAGCATTGT CCGAGAGGAC 240
   CAGACCCTGA AACCTGGCAA AGTCGATGTG AAGACAGATA AATGGGATTT CTACGAACAG 300
   TCGAC                                                             305

### (2) INFORMATION ZU SEQ ID NO: 9:

(i) SEQUENZ CHARAKTERISTIKA:
   (A) LÄNGE: 27 Basenpaare
   (B) ART: Nukleinsäure
   (C) STRANGFORM: Einzel
   (D) TOPOLOGIE: linear
(ii) ART DES MOLEKÜLS: cDNS
(xi) SEQUENZBESCHREIBUNG: SEQ ID NO: 9:
   GATGCATGCG GTCCTATGCC CAAGCTG 27

### (2) INFORMATION ZU SEQ ID NO: 10:

(i) SEQUENZ CHARAKTERISTIKA:
   (A) LÄNGE: 27 Basenpaare
   (B) ART: Nukleinsäure
   (C) STRANGFORM: Einzel
   (D) TOPOLOGIE: linear
(ii) ART DES MOLEKÜLS: cDNS
(xi) SEQUENZBESCHREIBUNG: SEQ ID NO: 10:
   GATAAGCTTT CACTGGCAGT AGAAATC 27

### (2) INFORMATION ZU SEQ ID NO: 11:

(i) SEQUENZ CHARAKTERISTIKA:
   (A) LÄNGE: 28 Basenpaare
   (B) ART: Nukleinsäure
   (C) STRANGFORM: Einzel
   (D) TOPOLOGIE: linear
(ii) ART DES MOLEKÜLS: cDNS
(xi) SEQUENZBESCHREIBUNG: SEQ ID NO: 11:
   GATCTAGCCG GCCGCCCAGC CCGGCATG 28

### (2) INFORMATION ZU SEQ ID NO: 12:

(i) SEQUENZ CHARAKTERISTIKA:
   (A) LÄNGE: 20 Basenpaare
   (B) ART: Nukleinsäure
   (C) STRANGFORM: Einzel
   (D) TOPOLOGIE: linear
(ii) ART DES MOLEKÜLS: cDNS
(xi) SEQUENZBESCHREIBUNG: SEQ ID NO: 12:
   CCGGGCTGGG CGGCCGGCTA 20

### (2) INFORMATION ZU SEQ ID NO: 13:

(i) SEQUENZ CHARAKTERISTIKA:
   (A) LÄNGE: 16 Aminosäuren
   (B) ART: Aminosäure
   (C) STRANGFORM: Einzel
   (D) TOPOLOGIE: linear
(ii) ART DES MOLEKÜLS: Peptid
(xi) SEQUENZBESCHREIBUNG: SEQ ID NO: 13:
   Met Arg Gly Ser His His His His His His Gly Ser Ser Arg Pro Pro
   1 5 10 15

### (2) INFORMATION ZU SEQ ID NO: 14:

(i) SEQUENZ CHARAKTERISTIKA:
   (A) LÄNGE: 18 Aminosäuren
   (B) ART: Aminosäure
   (C) STRANGFORM: Einzel
   (D) TOPOLOGIE: linear
(ii) ART DES MOLEKÜLS: Peptid
(xi) SEQUENZBESCHREIBUNG: SEQ ID NO: 14:
   Met Arg Gly Ser His His His His His His Gly Ser Val Asp Asp Asp
   1 5 10 15
   Asp Lys

### (2) INFORMATION ZU SEQ ID NO: 15:

(i) SEQUENZ CHARAKTERISTIKA:
   (A) LÄNGE: 43 Basenpaare
   (B) ART: Nukleinsäure
   (C) STANGFORM: Einzel
   (D) TOPOLOGIE: linear
(ii) ART DES MOLEKÜLS: cDNS
(xi) SEQUENZBESCHREIBUNG: SEQ ID NO: 15:
   AAAAAGGATC CAGCCGGCCG CCCGGTCCTA TGCCCAAGCT GGC 43

### (2) INFORMATION ZU SEQ ID NO: 16:

(i) SEQUENZ CHARAKTERISTIKA:
   (A) LÄNGE: 23 Basenpaare
   (B) ART: Nukleinsäure
   (C) STRANGFORM: Einzel
   (D) TOPOLOGIE: linear
(ii) ART DES MOLEKÜLS: cDNS
(xi) SEQUENZBESCHREIBUNG: SEQ ID NO: 16:
   GGCGAGCAGC CAGATCTCCA TAG 23

### (2) INFORMATION ZU SEQ ID NO: 17:

(i) SEQUENZ CHARAKTERISTIKA:
   (A) LÄNGE: 50 Basenpaare
   (B) ART: Nukleinsäure
   (C) STRANGFORM: Einzel
   (D) TOPOLOGIE: linear
(ii) ART DES MOLEKÜLS: cDNS
(xi) SEQUENZBESCHREIBUNG: SEQ ID NO: 17:
   AAAAAAGGAT CCGTTGATGA TGACGATAAA GGTCCTATGC CCAAGCTGGC 50

### (2) INFORMATION ZU SEQ ID NO: 18:

(i) SEQUENZ CHARAKTERISTIKA:
   (A) LÄNGE: 330 Basenpaare
   (B) ART: Nukleinsäure
   (C) STRANGFORM: Einzel
   (D) TOPOLOGIE: linear
(ii) ART DES MOLEKÜLS: cDNS
(ix) MERKMALE:
   (A) NAME/SCHLÜSSEL: mat_peptide
   (B) LAGE: 7..327
(ix) MERKMALE:
   (A) NAME/SCHLÜSSEL: misc_RNA
   (B) LAGE: 4..6
   (D) SONSTIGE ANGABEN: /function= "Startcodon Met"
(xi) SEQUENZBESCHREIBUNG: SEQ ID NO: 18:
   CATATGGGAC CAATGCCAAA ATTAGCAGAT CGTAAATTAT GTGCAGATCA GGAGTGCAGC    60
   CACCCTATCT CCATGGCTGT GGCCCTTCAG GACTACATGG CCCCCGACTG CCGATTCCTG   120
   ACCATTCACC GGGGCCAAGT GGTGTATGTC TTCTCCAAGC TGAAGGGCCG TGGGCGGCTC   180
   TTCTGGGGAG GCAGCGTTCA GGGAGATTAC TATGGAGATC TGGCTGCTCG CCTGGGCTAT   240
   TTCCCCAGTA GCATTGTCCG AGAGGACCAG ACCCTGAAAC CTGGCAAAGT CGATGTGAAG   300
   ACAGACAAAT GGGATTTCTA CTGCCAGTGA                                    330

### (2) INFORMATION ZU SEQ ID NO: 19:

(i) SEQUENZ CHARAKTERISTIKA:
   (A) LÄNGE: 59 Basenpaare
   (B) ART: Nukleinsäure
   (C) STRANGFORM: Einzel
   (D) TOPOLOGIE: linear
(ii) ART DES MOLEKÜLS: cDNS
(xi) SEQUENZBESCHREIBUNG: SEQ ID NO: 19:
   AAAAACATAT GGGACCAATG CCAAAATTAG CAGATCGTAA ATTATGTGCA GATCAGGAG    59

### (2) INFORMATION ZU SEQ ID NO: 20:

(i) SEQUENZ CHARAKTERISTIKA:
   (A) LÄNGE: 29 Basenpaare
   (B) ART: Nukleinsäure
   (C) STRANGFORM: Einzel
   (D) TOPOLOGIE: linear
(ii) ART DES MOLEKÜLS: cDNS
(xi) SEQUENZBESCHREIBUNG: SEQ ID NO: 20:
   AAAAAAAGCT TTCACTGGCA GTAGAAATC 29

### (2) INFORMATION ZU SEQ ID NO: 21:

(i) SEQUENZ CHARAKTERISTIKA:
   (A) LÄNGE: 33 Basenpaare
   (B) ART: Nukleinsäure
   (C) STRANGFORM: Einzel
   (D) TOPOLOGIE: linear
(ii) ART DES MOLEKÜLS: cDNS
(xi) SEQUENZBESCHREIBUNG: SEQ ID NO: 21:
   CGTGAATTCA ACATGGCCCG GTCCCTGGTG TGC 33

### (2) INFORMATION ZU SEQ ID NO: 22:

(i) SEQUENZ CHARAKTERISTIKA:
   (A) LÄNGE: 30 Basenpaare
   (B) ART: Nukleinsäure
   (C) STRANGFORM: Einzel
   (D) TOPOLOGIE: linear
(ii) ART DES MOLEKÜLS: cDNS
(xi) SEQUENZBESCHREIBUNG: SEQ ID NO: 22:
   TATCTGCAGT CACTGGCAGT AGAAATCCCA 30

### (2) INFORMATION ZU SEQ ID NO: 23:

(i) SEQUENZ CHARAKTERISTIKA:
   (A) LÄNGE: 260 Basenpaare
   (B) ART: Nukleinsäure
   (C) STRANGFORM: Einzel
   (D) TOPOLOGIE: linear
(ii) ART DES MOLEKÜLS: cDNS
(xi) SEQUENZBESCHREIBUNG: SEQ ID NO: 23:
   GTGGGAGGTC TATATAAGCA GAGCTCTCTG GCTAACTAGA GAACCCACTG CTTAACTGGC  60
   TTATCGAAAT TAATACGACT CACTATAGGG AGACCCAAGC TGTACCAGAT ATCAGGATCC 120
   CCCGGGCTGC AGGAATTCGA TATCAAGCTT CTCGAGGGGG GGCCCGGTAC CGATCCTGGC 180
   CAGCTAGCTA GTAGCTAGAG GATCTTTGTG AAGGAACCTT ACTTCTGTGG TGTGACATAA 240
   TTGGACAAAC TACCTACAGA                                             260

### (2) INFORMATION ZU SEQ ID NO: 24:

(i) SEQUENZ CHARAKTERISTIKA:
   (A) LÄNGE: 596 Basenpaare
   (B) ART: Nukleinsäure
   (C) STRANGFORM: Einzel
   (D) TOPOLOGIE: linear
(ii) ART DES MOLEKÜLS: DNS (genomisch)
(ix) MERKMALE:
   (A) NAME/SCHLÜSSEL: CDS
   (B) LAGE: join(40..111, 40..166, 214..347, 393..503, 549 ..569)
(ix) MERKMALE:
   (A) NAME/SCHLÜSSEL: sig_peptide
   (B) LAGE: 40..111
(ix) MERKMALE:
   (A) NAME/SCHLÜSSEL: exon
   (B) LAGE: 40..166
(ix) MERKMALE:
   (A) NAME/SCHLÜSSEL: exon
   (B) LAGE: 214..347
(ix) MERKMALE:
   (A) NAME/SCHLÜSSEL: exon
   (B) LAGE: 393..503
(ix) MERKMALE:
   (A) NAME/SCHÜSSEL: exon
   (B) LAGE: 549..569
(ix) MERKMALE:
   (A) NAME/SCHÜSSEL: -
   (B) LAGE: one-of(194, 369, 527)
   (D) SONSTIGE ANGABEN: /note= "N in den Positionen 194, 369 und 527 steht fuer eine unbestimmte Anzahl und Sequenz von Nukleotiden"
(xi) SEQUENZBESCHREIBUNG: SEQ ID NO: 24:
   CCAGCACCCC CTTGCTCACT CTCTTGCTCA CAGTCCACGA TGGCCCGGTC CCTGGTGTGC   60
   CTTGGTGTCA TCATCTTGCT GTCTGCCTTC TCCGGACCTG GTGTCAGGGG TGGTCCTATG  120
   CCCAAGCTGG CTGACCGGAA GCTGTGTGCG GACCAGGAGT GCAGCCGTAA GAATGGGGAG  180
   GGTAGAATTG GGNCCCTTCT ATTCCTTCCC TAGACCCTAT CTCCATGGCT GTGGCCCTTC  240
   AGGACTACAT GGCCCCCGAC TGCCGATTCC TGACCATTCA CCGGGGCCAA GTGGTGTATG  300
   TCTTCTCCAA GCTGAAGGGC CGTGGGCGGC TCTTCTGGGG AGGCAGCGTG GGTCTTGGGA  360
   GAGTGAAANA GCTTTTAACT CCTCTTCCCC AGGTTCAGGG AGATTACTAT GGAGATCTGG  420
   CTGCTCGCCT GGGCTATTTC CCCAGTAGCA TTGTCCGAGA GGACCAGACC CTGAAACCTG  480
   GCAAAGTCGA TGTGAAGACA GACGTGGAGT GTCATGGGGG CTGGCANTTT CCCCTTTCTC  540
   TTTTTCAGAA ATGGGATTTC TACTGCCAGT GAGCTCAGCC TACCGCTGGC CCTGCC       596